# EUROPEAN PATENT SPECIFICATION

(11) **EP 4 095 124 B1**
(45) Date of publication and mention of the grant of the patent: **24.12.2025**
(21) Application number: 21744716.8
(22) Date of filing: 20.01.2021
(51) Int. Cl.: C07C 211/54, H10K 85/60, C07C 211/61, H10K 50/18

(54) **ARYLAMINE COMPOUND AND ORGANIC ELECTROLUMINESCENT ELEMENT**
ARYLAMINVERBINDUNG UND ORGANISCHES ELEKTROLUMINESZENZELEMENT
COMPOSÉ ARYLAMINE ET ÉLÉMENT ÉLECTROLUMINESCENT ORGANIQUE

(30) Priority: 21.01.2020 JP 2020007670
(43) Date of publication of application: 30.11.2022
(73) Proprietor: Hodogaya Chemical Co., Ltd., Tokyo, 105-0021 (JP)
(72) Inventor: KASE, Kouki, Tokyo 104-0028 (JP); YAMAMOTO, Takeshi, Tokyo 104-0028 (JP); LIM, Jae-Geon, Tokyo 104-0028 (JP); IZUMIDA, Junichi, Tokyo 104-0028 (JP); HAYASHI, Shuichi, Tokyo 104-0028 (JP); YOKOYAMA, Norimasa, Tokyo 104-0028 (JP)
(74) Representative: Forstmeyer, Dietmar
(86) International application number: PCT/JP2021/001899
(87) International publication number: WO 2021/149735

(56) References cited:
- WO-A1-2019/098234
- WO-A1-2019/098234
- WO-A2-2011/002870
- JP-A- 2003 048 868
- JP-A- 2003 048 868
- JP-A- 2009 170 810
- JP-A- 2009 170 810
- JP-A- 2012 532 111

## Description

### Technical Field

The present invention relates to a compound suitable for an organic electroluminescence device (abbreviated hereinbelow as "organic EL device") which is a self-luminous light-emitting device suitable for various display devices, and to such an organic EL device. More specifically, the present invention relates to an arylamine compound, and to an organic EL device employing the compound.

### Background Art

Organic EL devices, which are self-luminous light-emitting devices, are brighter, have better visibility, and are capable of clearer display compared to liquid crystal devices. Therefore, organic EL devices have been actively researched.

In 1987, C. W. Tang et al. of Eastman Kodak Company developed a device having a multilayer structure wherein various roles for light emission were allotted respectively to different materials, thereby achieving practical utilization of organic EL devices using organic materials. They developed a laminate including a layer of a fluorescent substance capable of transporting electrons and a layer of an organic substance capable of transporting holes. Injecting both charges into the fluorescent substance layer causes emission of light, achieving a high luminance of 1,000 cd/m² or higher at a voltage of 10 V or less (see, for example, Patent Literatures 1 and 2).

Many improvements have been heretofore made for practical utilization of organic EL devices. Further subdivision of the various roles within the multilayer structure has yielded an electroluminescent device wherein an anode, a hole injection layer, a hole transport layer, a light-emitting layer, an electron transport layer, an electron injection layer, and a cathode are formed sequentially on a substrate, achieving high efficiency and high durability (see, for example, Non-Patent Literature 1).

To further improve luminous efficiency, attempts have been made to employ triplet excitons. Also, the use of phosphorescent compounds has been investigated (see, for example, Non-Patent Literature 2). Further, devices employing light emission by thermally activated delayed fluorescence (TADF) have been developed. In 2011, Adachi et al. of Kyushu University achieved an external quantum efficiency of 5.3% with a device using a thermally activated delayed fluorescence material (see, for example, Non-Patent Literature 3).

Alight-emitting layer can be prepared by doping a charge-transporting compound, typically called a "host material", with a fluorescent compound, a phosphorescent compound, or a material emitting delayed fluorescence. As described in the aforementioned Non-Patent Literatures, the selection of organic materials in an organic EL device greatly affects various properties such as efficiency and durability of that device (see, for example, Non-Patent Literature 2).

In organic EL devices, charges injected from the respective electrodes recombine in the light-emitting layer to emit light. Thus, what is important is to efficiently deliver the charges, i.e., the holes and electrons, to the light-emitting layer, which requires the device to have excellent carrier balance. So, by using a material having properties of enhancing hole injectability for supplying the holes injected from the anode to the light-emitting layer and also enhancing electron blockability for blocking the electrons injected from the cathode, it is possible to increase the probability of hole-electron recombination within the light-emitting layer. Also, by confining excitons generated within the light-emitting layer, high luminous efficiency can be achieved. Therefore, the hole-transporting material serves an important role, and there is thus a demand for a hole-transporting material having high hole injectability, high hole mobility, high electron blockability, and high durability against electrons.

In terms of device longevity, the material's heat resistance and amorphous properties are also important. With a material having poor heat resistance, thermal decomposition and material degradation occur, even at low temperatures caused by heat produced when the device is driven. With a material having poor amorphous properties, a thin film undergoes crystallization in a short time, resulting in device degradation. Thus, the material to be used requires high heat resistance and good amorphous properties.

Examples of hole-transporting materials used heretofore in organic EL devices include N,N'-diphenyl-N,N'-di(α-naphthyl)-benzidine ("NPD") and various aromatic amine derivatives (see, for example, Patent Literatures 1 and 2). NPD does have good hole transportability, but its glass transition point (Tg), serving as an index of heat resistance, is as low as 96°. This may cause degradation in device properties due to crystallization in high-temperature conditions (see, for example, Non-Patent Literature 4).

Among the aromatic amine derivatives disclosed in the aforementioned Patent Literatures, there are compounds having excellent mobility, such as hole mobility of 10⁻³ cm²/Vs or greater (see, for example, Patent Literatures 1 and 2). These compounds, however, have insufficient electron blockability, thus allowing a portion of the electrons to pass through the light-emitting layer and thereby making it impossible to expect improvements in luminous efficiency. To achieve even higher efficiency, there is a demand for a material having higher electron blockability and higher heat resistance and providing a more stable thin film. Further, aromatic amine derivatives having high durability have been reported (see, for example, Patent Literature 3), but these compounds are used as charge-transporting materials to be used in electrophotographic photoreceptors, and have never been used in organic EL devices.

To overcome these drawbacks, arylamine compounds having a substituted carbazole structure or a triarylbenzene structure have been proposed as compounds having improved properties such as heat resistance and hole injectability (see, for example, Patent Literatures 4 and 5). Devices using these compounds for the hole injection layer or hole transport layer are improved in terms of device life and luminous efficiency, but these improvements are still insufficient, and there are further demands for even lower driving voltage, higher luminous efficiency, and longer device life.

### Citation List

### Patent Literature

Patent Literature 1: US5792557 (A)
Patent Literature 2: US5639914 (A)
Patent Literature 3: US7759030 (B2)
Patent Literature 4: US8021764 (B2)
Patent Literature 5: US20180175301 (A1)
Patent Literature 6: EP2684932 (A1)

### Non-Patent Literature

Non-Patent Literature 1: Japan Society of Applied Physics, 9th Class, Proceedings, pp. 55-61 (2001)
Non-Patent Literature 2: Japan Society of Applied Physics, 9th Class, Proceedings, pp. 23-31 (2001)
Non-Patent Literature 3: Appl. Phys. Let., 98, 083302 (2011)
Non-Patent Literature 4: Japan OLED Forum, Collection of Abstracts for 3rd Regular Meeting, pp. 13-14 (2006)

WO2019098234 (A1) discloses arylamine compounds suitable to be used in OLED devices.

### Summary of Invention

### Technical Problem

An objective of the present invention is to provide, as a material for highly-efficient highly-durable organic EL devices, a material for organic EL devices which has: (1) excellent hole injectability and transportability; (2) electron blockability; (3) high stability in a thin-film state; and (4) excellent durability.

Another objective of the present invention is to provide, by using the present material, an organic EL device which has: (1) high luminous efficiency and power efficiency; (2) a low emission start voltage and practical driving voltage; and (3) a long lifetime.

### Solution to Problem

To achieve the aforementioned objectives, Inventors focused on the fact that arylamine compounds having a triarylbenzene structure have excellent hole injectability and transportability, thin film stability, and durability, and diligently studied these compounds to pursue optimization and improvement in substitution positions, thereby achieving a material having dramatically improved properties. Inventors have also found that the use of this material in an organic EL device can improve performance in luminous efficiency and power efficiency, suppress emission start voltage and practical driving voltage, and achieve longer lifetime surpassing conventional lifetime, to thus accomplish the present invention.

The present invention is an aryl compound selected from the following compounds: 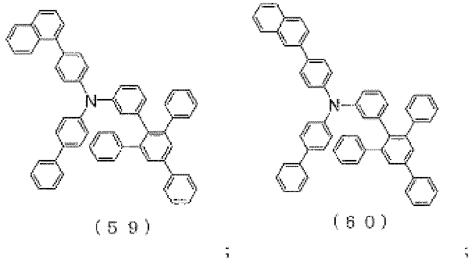 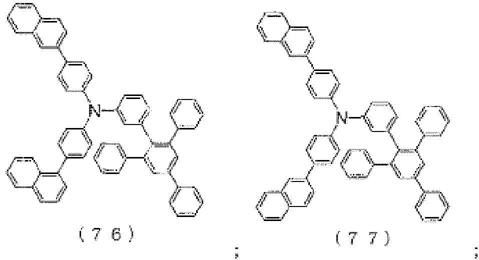 and 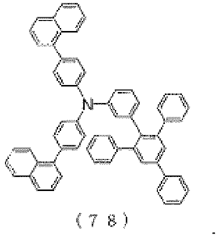
{1} An arylamine compound not forming part of the present invention may be represented by general formula (1) below.

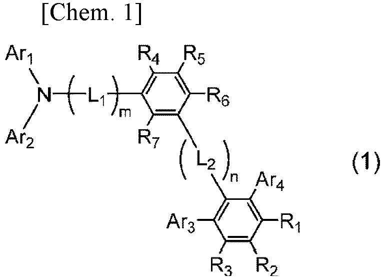

(In the formula, Ar₁, Ar₂, Ar₃, and Ar₄ may be the same or different from one another, and each represent a substituted or unsubstituted aromatic hydrocarbon group, a substituted or unsubstituted aromatic heterocyclic group, or a substituted or unsubstituted fused polycyclic aromatic group;
L₁ and L₂ may be the same or different from one another, and each represent a divalent substituted or unsubstituted aromatic hydrocarbon group, a divalent substituted or unsubstituted aromatic heterocyclic group, or a divalent substituted or unsubstituted fused polycyclic aromatic group;
R₁ to R₇ may be the same or different from one another, and each represent a hydrogen atom, a deuterium atom, a fluorine atom, a chlorine atom, a cyano group, a nitro group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group; and
m and n may be the same or different from one another, and each represent an integer from 0 to 2, wherein L₁ represents a single bond when m is 0, and L₂ represents a single bond when n is 0.)

Concrete examples of the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by Ar₁ to Ar₄ in the general formula (1) may include, for example, aryl groups having 6 to 30 carbon atoms and hetero aryl groups having 2 to 20 carbon atoms, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc.

Concrete examples of the "substituent" in the "substituted aromatic hydrocarbon group", "substituted aromatic heterocyclic group" or "substituted fused polycyclic aromatic group" as represented by Ar₁ to Ar₄ in the general formula (1) may include: a deuterium atom; a cyano group; a nitro group; halogen atoms, such as a fluorine atom, a chlorine atom, a bromine atom, or an iodine atom; silyl groups, such as a trimethylsilyl group, triphenylsilyl group, etc.; linear or branched alkyl groups having 1 to 6 carbon atoms, such as a methyl group, an ethyl group, a propyl group, etc.; linear or branched alkyloxy groups having 1 to 6 carbon atoms, such as a methyloxy group, an ethyloxy group, a propyloxy group, etc.; alkenyl groups, such as a vinyl group, an allyl group, etc.; aryloxy groups, such as a phenyloxy group, a tolyloxy group, etc.; arylalkyloxy groups, such as a benzyloxy group, a phenethyloxy group, etc.; aromatic hydrocarbon groups or fused polycyclic aromatic groups, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, etc.; and aromatic heterocyclic groups, such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, etc. These substituents may further be substituted by any of the substituents given as examples above. Further, the aforementioned substituent(s) and the substituted benzene ring, or a plurality of substituents substituting the same benzene ring, may be bonded to each other via a single bond, a substituted or unsubstituted methylene group, an oxygen atom, or a sulfur atom, to form a ring.

Examples of the "divalent aromatic hydrocarbon group", "divalent aromatic heterocyclic group" or "divalent fused polycyclic aromatic group" in the "divalent substituted or unsubstituted aromatic hydrocarbon group", "divalent substituted or unsubstituted aromatic heterocyclic group" or "divalent substituted or unsubstituted fused polycyclic aromatic group" as represented by L₁ and L₂ in the general formula (1) may include groups similar to divalent groups obtained by removing one hydrogen atom from the aforementioned examples given for the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by Ar₁ to Ar₄ in the general formula (1).

Examples of the "substituent" in the "substituted or unsubstituted aromatic hydrocarbon group", "substituted or unsubstituted aromatic heterocyclic group" or "substituted or unsubstituted fused polycyclic aromatic group" as represented by L₁ and L₂ in the general formula (1) may include the aforementioned examples given for the "substituent" in the "substituted aromatic hydrocarbon group", "substituted aromatic heterocyclic group" or "substituted fused polycyclic aromatic group" as represented by Ar₁ to Ar₄ in the general formula (1), and they may take similar forms/configurations as those described above.

Concrete examples of the "linear or branched alkyl group having 1 to 6 carbon atoms", "cycloalkyl group having 5 to 10 carbon atoms" or "linear or branched alkenyl group having 2 to 6 carbon atoms" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R₁ to R₇ in the general formula (1) may include a methyl group, an ethyl group, a n-propyl group, an isopropyl group, a n-butyl group, an isobutyl group, a tert-butyl group, a n-pentyl group, an isopentyl group, a neopentyl group, a n-hexyl group, a cyclopentyl group, a cyclohexyl group, a 1-adamantyl group, a 2-adamantyl group, a vinyl group, an allyl group, an isopropenyl group, a 2-butenyl group, etc. The aforementioned group(s) and the substituted benzene ring, or a plurality of these groups substituting the same benzene ring, may be bonded to each other via a single bond, a substituted or unsubstituted methylene group, a substituted or unsubstituted amino group, an oxygen atom, or a sulfur atom, to form a ring.

Examples of the "substituent" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R₁ to R₇ in the general formula (1) may include the aforementioned examples given for the "substituent" in the "substituted aromatic hydrocarbon group", "substituted aromatic heterocyclic group" or "substituted fused polycyclic aromatic group" as represented by Ar₁ to Ar₄ in the general formula (1), and they may take similar forms/configurations as those described above.

Concrete examples of the "carbon atom having a linear or branched alkyloxy group having 1 to 6 carbon atoms" or "carbon atom having a cycloalkyloxy group having 5 to 10 carbon atoms" in the "carbon atom having a linear or branched alkyloxy group having 1 to 6 carbon atoms and optionally having a substituent" or "carbon atom having a cycloalkyloxy group having 5 to 10 carbon atoms and optionally having a substituent" as represented by R₁ to R₇ in the general formula (1) may include a methyloxy group, an ethyloxy group, a n-propyloxy group, an isopropyloxy group, a n-butyloxy group, a tert-butyloxy group, a n-pentyloxy group, a n-hexyloxy group, a cyclopentyloxy group, a cyclohexyloxy group, a cycloheptyloxy group, a cyclooctyloxy group, a 1-adamantyloxy group, a 2-adamantyloxy group, etc. The aforementioned group(s) and the substituted benzene ring, or a plurality of these groups substituting the same benzene ring, may be bonded to each other via a single bond, a substituted or unsubstituted methylene group, a substituted or unsubstituted amino group, an oxygen atom, or a sulfur atom, to form a ring.

Examples of the "substituent" in the "linear or branched alkyloxy group having 1 to 6 carbon atoms and optionally having a substituent" or "cycloalkyloxy group having 5 to 10 carbon atoms and optionally having a substituent" as represented by R₁ to R₇ in the general formula (1) may include the aforementioned examples given for the "substituent" in the "substituted aromatic hydrocarbon group", "substituted aromatic heterocyclic group" or "substituted fused polycyclic aromatic group" as represented by Ar₁ to Ar₄ in the general formula (1), and they may take similar forms/configurations as those described above.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by R₁ to R₇ in the general formula (1) may include the aforementioned examples given for the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by Ar₁ to Ar₄ in the general formula (1).

Examples of the "substituent" in the "substituted or unsubstituted aromatic hydrocarbon group", "substituted or unsubstituted aromatic heterocyclic group" or "substituted or unsubstituted fused polycyclic aromatic group" as represented by R₁ to R₇ in the general formula (1) may include the aforementioned examples given for the "substituent" in the "substituted aromatic hydrocarbon group", "substituted aromatic heterocyclic group" or "substituted fused polycyclic aromatic group" as represented by Ar₁ to Ar₄ in the general formula (1), and they may take similar forms/configurations as those described above.

Ar₁ to Ar₄ in the general formula (1) may preferably be a phenyl group, a biphenyl group, a naphthyl group, a phenanthrenyl group, a fluorenyl group or a spirobifluorenyl group, which may be substituted or unsubstituted, and more preferably a phenyl group, a biphenyl group, a naphthyl group or a phenanthrenyl group, which may be substituted or unsubstituted. Ar₃ and Ar₄ may preferably be a phenyl group or a naphthyl group, which may be substituted or unsubstituted, and more preferably, Ar₃ and Ar₄ are the same. Particularly, Ar₁ and Ar₂ may preferably be a phenyl group, a biphenyl group, a naphthyl group or a phenanthrenyl group, or a group having a structure in which two groups selected from the above are bonded, and Ar₃ and Ar₄ may preferably be a phenyl group.

The sum of integers m and n in the general formula (1) may preferably be 0 or 1, and more preferably, m may be 0 or 1, and n may be 0. Examples of L₁ and L₂ in the general formula (1) wherein m and n are not 0 may preferably be a phenylene group, a biphenylene group or a naphthalene group, which may be substituted or unsubstituted, and more preferably an unsubstituted phenylene group or naphthalene group.

R₁ to R₇ in the general formula (1) may preferably be a hydrogen atom or a deuterium atom, or a phenyl group, a biphenyl group, a naphthyl group, an anthracenyl group, a phenanthrenyl group, a fluorenyl group or a spirobifluorenyl group, which may be substituted or unsubstituted, and more preferably a hydrogen atom, a deuterium atom, or an unsubstituted phenyl group, biphenyl group or naphthyl group. R₁ and R₃ may preferably be a hydrogen atom or a deuterium atom, and R₂ may preferably be a hydrogen atom or a deuterium atom, or a phenyl group, a biphenylyl group, a naphthyl group, a phenanthrenyl group or a fluorenyl group, which may be substituted or unsubstituted. Ar₃, Ar₄, and R₂ are preferably the same. Particularly, R₁, R₃, R₄, R₅, R₆, and R₇ may preferably be a hydrogen atom or a deuterium atom, and R₂ may preferably be a phenyl group.

The arylamine compound represented by the aforementioned general formula (1), which is suitably usable for an organic EL device, can preferably be used as a constituent material of a hole injection layer, a hole transport layer, an electron blocking layer, or a light-emitting layer of the organic EL device, and can more preferably be used as a constituent material of a hole transport layer or an electron blocking layer.

### Advantageous Effects of Invention

Compared to conventional hole-transporting materials, the arylamine compound of the present invention has various properties, such as (1) good hole injectability, (2) high hole mobility, (3) excellent electron blockability, (4) high electron tolerance, (5) stability in a thin-film state, and (6) excellent heat resistance. By using the arylamine compound of the present invention in an organic EL device, various properties can be achieved, such as (7) high luminous efficiency, (8) a low emission start voltage, (9) a low practical driving voltage, and (10) a long lifetime.

The arylamine compound of the present invention has excellent hole injectability and transportability, thin film stability, and durability. Thus, an organic EL device having a hole injection layer and/or hole transport layer produced by using the present compound as a hole-inj ecting material and/or hole-transporting material is improved in hole-transporting efficiency to the light-emitting layer and is thus improved in luminous efficiency, and also has a reduced driving voltage and is thereby improved in device durability. Thus, properties such as high efficiency, low driving voltage, and long lifetime can be achieved.

The arylamine compound of the present invention is characterized by having excellent electron blockability and high electron tolerance, being stable in a thin-film state, and being capable of confining excitons produced in the light-emitting layer. Thus, an organic EL device having an electron blocking layer produced by using the present compound as an electron blocking material has a higher probability of electron-hole recombination, is reduced in thermal inactivation, and thus has high luminous efficiency. Further, the organic EL device is reduced in driving voltage and improved in current resistance, and thereby improved in maximum light emission luminance.

The arylamine compound of the present invention has excellent hole transportability and a wide band gap. Thus, in an organic EL device having a light-emitting layer produced using this compound as a host material, by making the light-emitting layer support a dopant, e.g., a fluorescent substance, a phosphorescent substance, or a delayed fluorescent substance, the driving voltage is reduced and luminous efficiency is improved.

As described above, the arylamine compound of the present invention is useful as a material for a hole injection layer, a hole transport layer, an electron blocking layer, or a light-emitting layer of an organic EL device, and can improve luminous efficiency, driving voltage, and durability of conventional organic EL devices.

The arylamine compound of the present invention is not only usable for organic EL devices, but also usable in the field of electronic devices, such as electrophotographic photoreceptors, image sensors, photoelectric conversion devices, solar cells, etc.

### Brief Description of Drawings

[Fig. 1] Fig. 1 is a diagram illustrating Compounds (1) to (12) as preferable concrete examples of arylamine compounds represented by general formula (1).
[Fig. 2] Fig. 2 is a diagram illustrating Compounds (13) to (24) as preferable concrete examples of arylamine compounds represented by general formula (1).
[Fig. 3] Fig. 3 is a diagram illustrating Compounds (25) to (36) as preferable concrete examples of arylamine compounds represented by general formula (1).
[Fig. 4] Fig. 4 is a diagram illustrating Compounds (37) to (48) as preferable concrete examples of arylamine compounds represented by general formula (1).
[Fig. 5] Fig. 5 is a diagram illustrating Compounds (49) to (60) as preferable concrete examples of arylamine compounds represented by general formula (1).
[Fig. 6] Fig. 6 is a diagram illustrating Compounds (61) to (72) as preferable concrete examples of arylamine compounds represented by general formula (1).
[Fig. 7] Fig. 7 is a diagram illustrating Compounds (73) to (84) as preferable concrete examples of arylamine compounds represented by general formula (1).
[Fig. 8] Fig. 8 is a diagram illustrating Compounds (85) to (96) as preferable concrete examples of arylamine compounds represented by general formula (1).
[Fig. 9] Fig. 9 is a diagram illustrating Compounds (97) to (108) as preferable concrete examples of arylamine compounds represented by general formula (1).
[Fig. 10] Fig. 10 is a diagram illustrating Compounds (109) to (120) as preferable concrete examples of arylamine compounds represented by general formula (1).
[Fig. 11] Fig. 11 is a diagram illustrating Compounds (121) to (132) as preferable concrete examples of arylamine compounds represented by general formula (1).
[Fig. 12] Fig. 12 is a diagram illustrating Compounds (133) to (144) as preferable concrete examples of arylamine compounds represented by general formula (1).
[Fig. 13] Fig. 13 is a diagram illustrating Compounds (145) to (153) as preferable concrete examples of arylamine compounds represented by general formula (1).
[Fig. 14] Fig. 14 is a diagram illustrating a configuration of an organic EL device for Examples 32 to 60 and Comparative Examples 1 and 2.

### Description of Embodiments

The arylamine compounds of the present invention are novel compounds, but these compounds can be synthesized according to known methods (see, for example, Patent Literature 5).

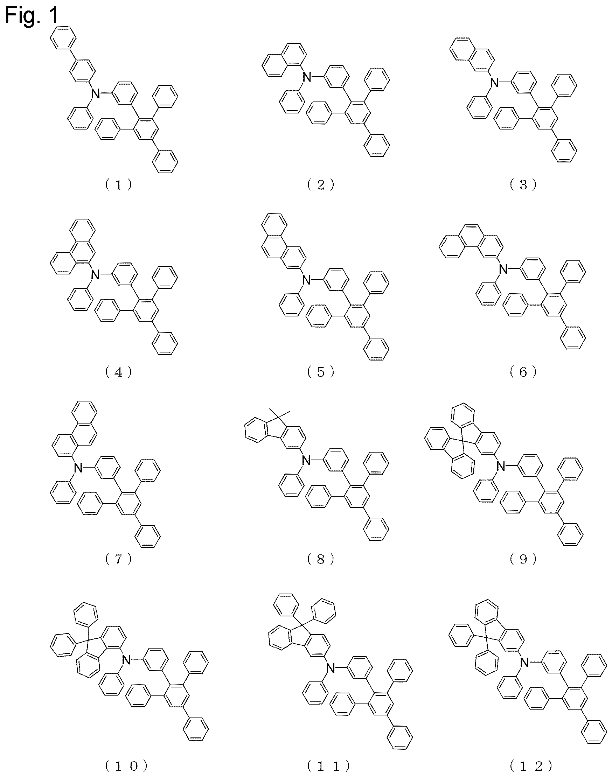

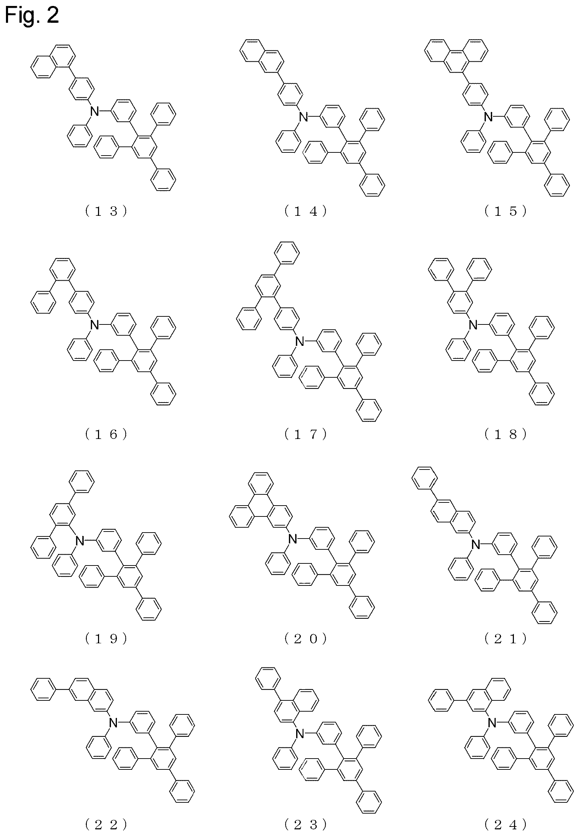

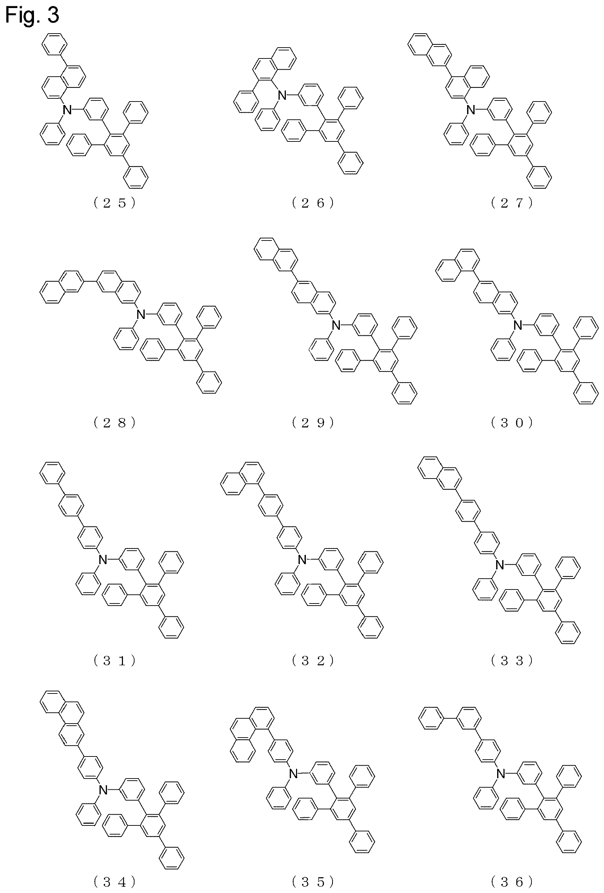

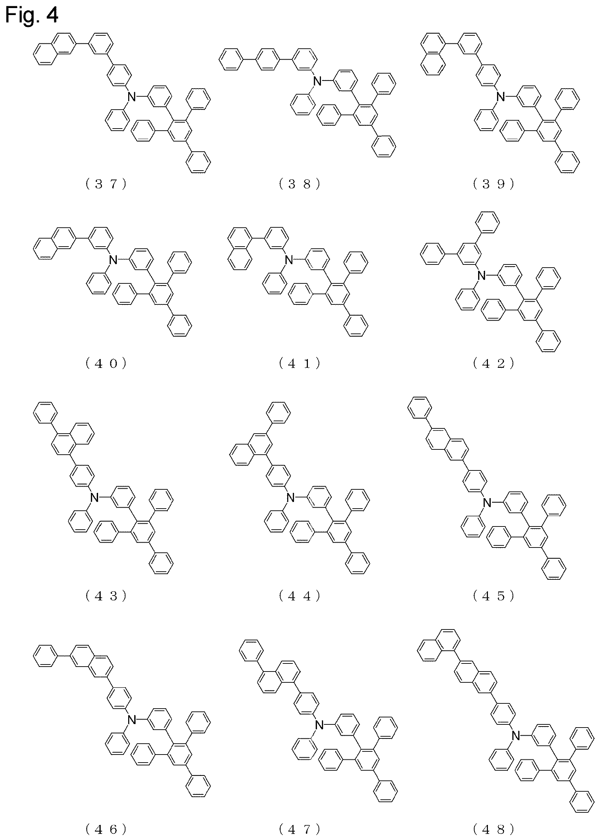

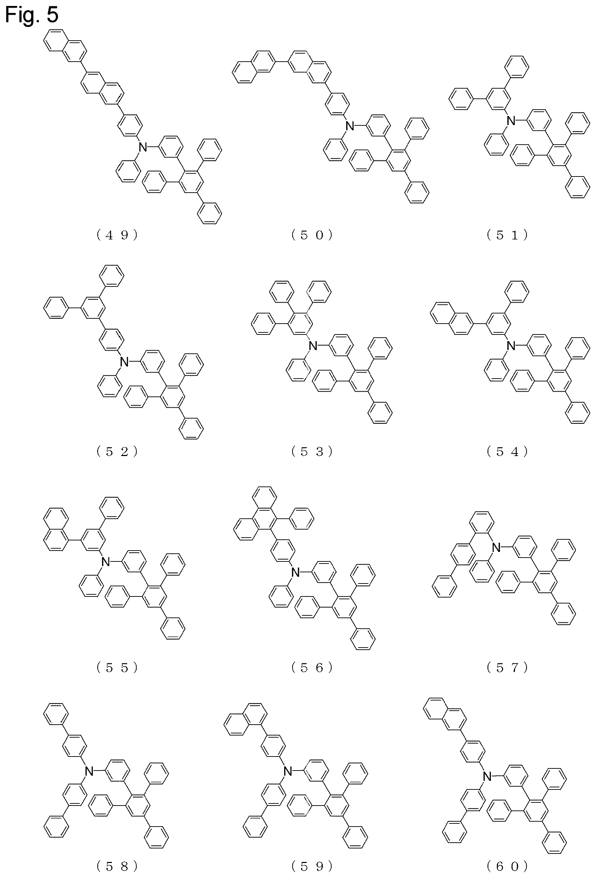

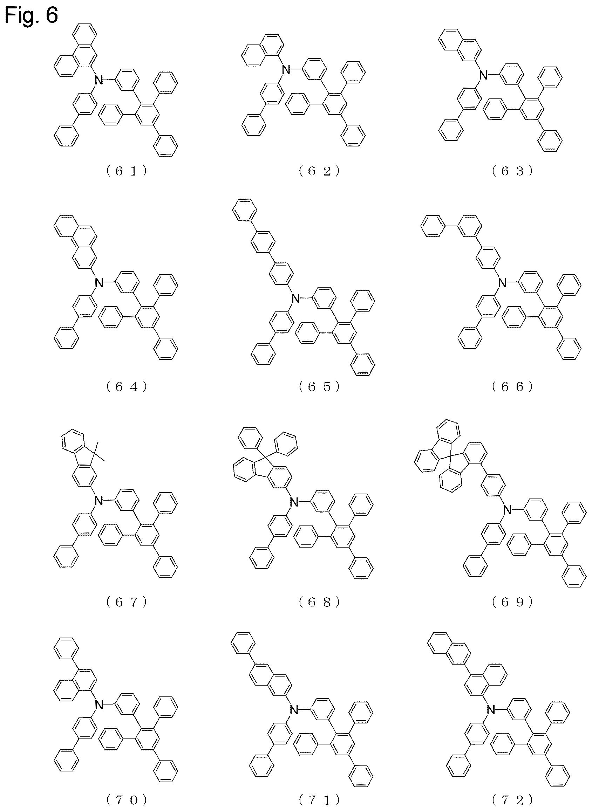

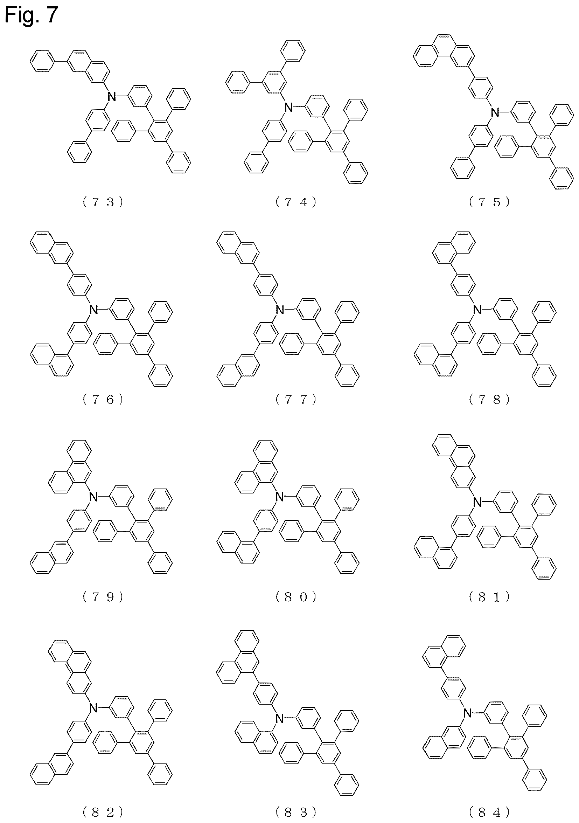

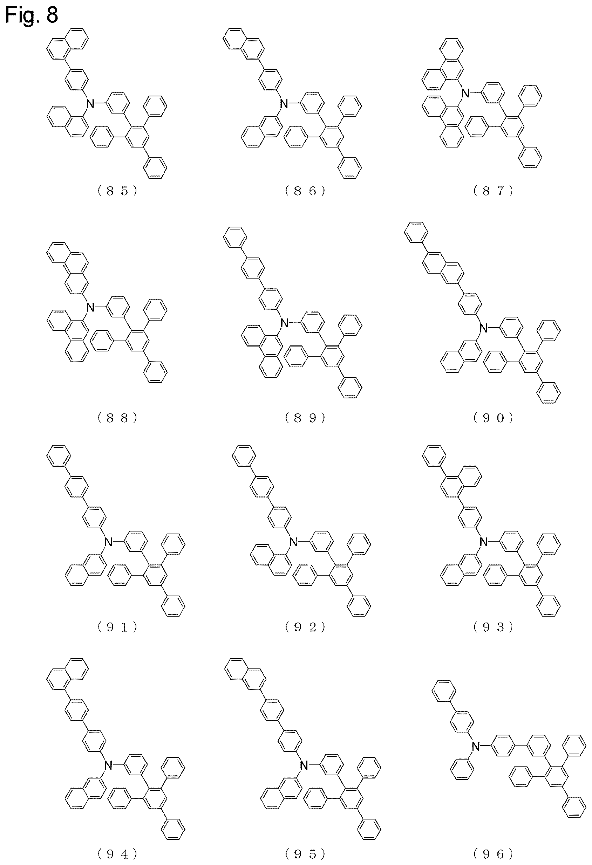

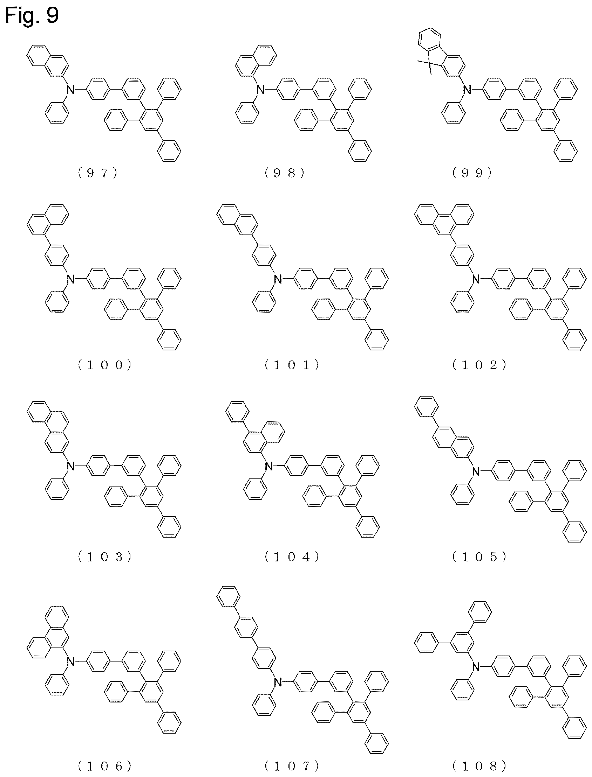

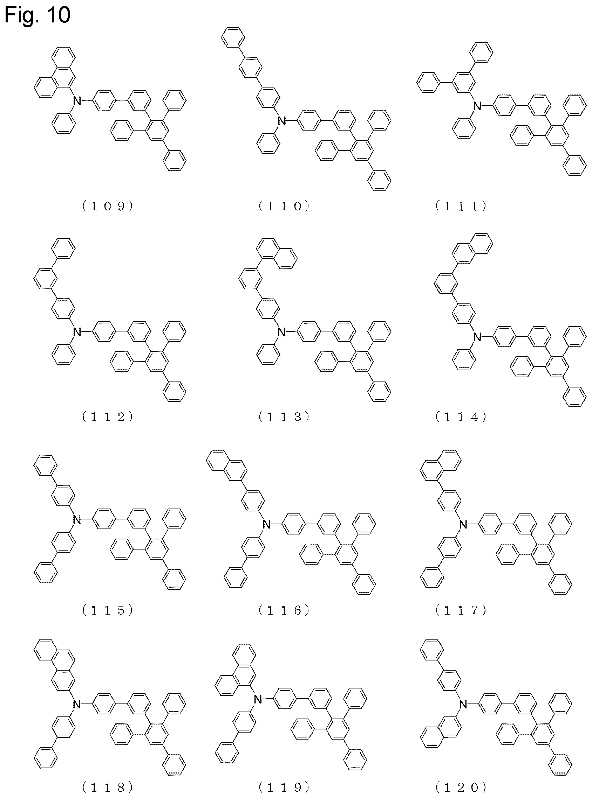

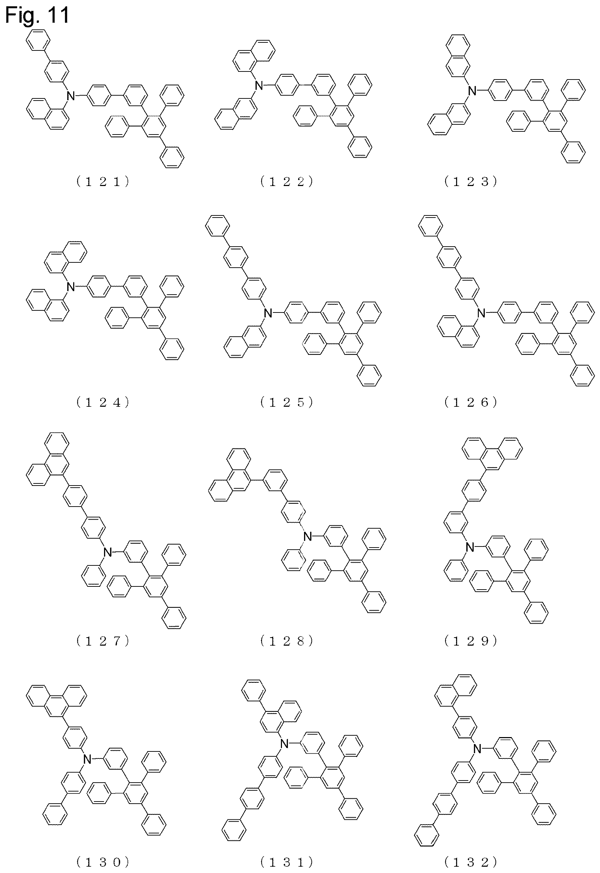

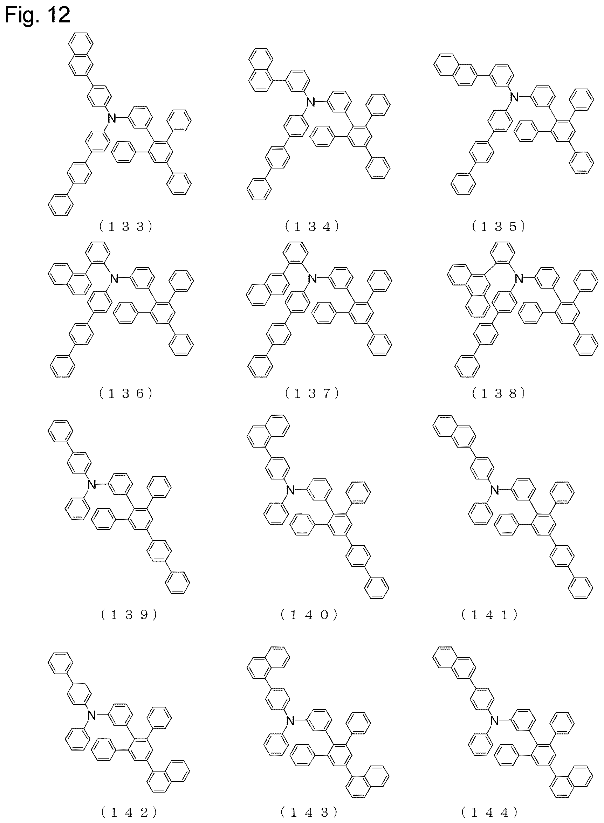

Figs. 1 to 12 illustrate concrete examples of preferred compounds among arylamine compounds represented by the general formula (1) that may be suitably used for the organic EL device. Note, however, that the compounds are not limited to the illustrated compounds. The compounds of the present claim 1 fall into the definition of the compounds represented by the general formula (1).

The arylamine compound represented by general formula (1) can be purified by known methods, such as column chromatography purification, adsorption purification with silica gel, activated carbon, activated clay, etc., recrystallization or crystallization using a solvent, sublimation purification, or the like. Compound identification can be achieved by NMR analysis. Physical properties to be measured may include such values as the melting point, glass transition point (Tg), work function, or the like. The melting point serves as an index of vapor deposition characteristics. The glass transition point (Tg) serves as an index of stability in a thin-film state. The work function serves as an index of hole injectability, hole transportability, or electron blockability.

The melting point and glass transition point (Tg) can be measured, for example, with a high-sensitivity differential scanning calorimeter (DSC3100SA from Bruker AXS) using a powder.

The work function can be found, for example, with an ionization potential measurement device (PYS-202 from Sumitomo Heavy Industries, Ltd.) by preparing a 100-nm thin film on an ITO substrate.

A structure of the organic EL device of the present invention may, for example, sequentially include, on a substrate, an anode, a hole injection layer, a hole transport layer, a light-emitting layer, an electron transport layer, an electron injection layer, and a cathode. In other examples, an electron blocking layer may be provided between the hole transport layer and the light-emitting layer, or a hole blocking layer may be provided between the light-emitting layer and the electron transport layer. In such multilayer structures, a single organic layer may have functions of several layers; for example, a single organic layer may have functions of the hole injection layer and the hole transport layer, or functions of the electron injection layer and the electron transport layer. Further, it is possible to stack two or more organic layers having the same function; for example, the structure may include: two stacked hole transport layers; two stacked light-emitting layers; or two stacked electron transport layers.

For the anode in the organic EL device of the present invention, it is possible to use an electrode material having a large work function, such as ITO or gold. For the material for the hole injection layer in the organic EL device of the present invention, it is possible to use: a porphyrin compound typified by copper phthalocyanine; a starburst triphenylamine derivative; an arylamine compound including, in its molecule, two or more triphenylamine structures or carbazolyl structures which are linked by a single bond or a divalent group containing no hetero atom; an acceptor heterocyclic compound such as hexacyanoazatriphenylene; or a coating-type polymer material. These materials can form thin films by known methods, such as vapor deposition, spin coating, ink-jetting, etc.

For the material for the hole injection layer and the hole transport layer in the organic EL device of the present invention, it is possible to use, other than the arylamine compound of the present invention, a benzidine derivative, such as N,N'-diphenyl-N,N'-di(m-tolyl)-benzidine (abbreviated hereinbelow as "TPD"), N,N'-diphenyl-N,N'-di(α-naphthyl)-benzidine (abbreviated hereinbelow as "NPD"), N,N,N',N'-tetrabiphenylylbenzidine, etc., or 1,1-bis[(di-4-tolylamino)phenyl]cyclohexane (abbreviated hereinbelow as "TAPC"), or an arylamine compound including, in its molecule, two or more triphenylamine structures or carbazolyl structures which are linked by a single bond or a divalent group containing no hetero atom. These materials may each be formed into a film singly, or a plurality of types of these materials may be mixed and formed into a film, and each may be used as a single layer. It is possible to form a laminate structure constituted by layers each formed singly by the respective materials, or a laminate structure constituted by layers formed by mixing, or a laminate structure constituted by layers each formed singly by the respective materials and layers formed by mixing a plurality of types of materials. Further, for the material for the hole injection-transport layer, it is possible to use a coating-type polymer material, such as poly(3,4-ethylenedioxythiophene) (abbreviated hereinbelow as "PEDOT")/poly(styrene sulfonate) (abbreviated hereinbelow as "PSS"). These materials can form thin films by known methods, such as vapor deposition, spin coating, ink-jetting, etc.

For the hole injection layer or the hole transport layer, it is possible to use: a material ordinarily used for such layers and p-doped with, for example, trisbromophenylamine hexachloroantimonate or a radialene derivative (see, for example, Patent Literature 6); or a polymer compound having, as a partial structure thereof, a benzidine derivative structure such as TPD.

For the material for the electron blocking layer in the organic EL device of the present invention, it is possible to use, other than the arylamine compound of the present invention, a compound having an electron blocking action, such as: a carbazole derivative, such as 4,4',4"-tri(N-carbazolyl)triphenylamine (abbreviated hereinbelow as "TCTA"), 9,9-bis[4-(carbazol-9-yl)phenyl]fluorene, 1,3-bis(carbazol-9-yl)benzene (abbreviated hereinbelow as "mCP"), 2,2-bis(4-carbazol-9-ylphenyl)adamantane (abbreviated hereinbelow as "Ad-Cz"), etc.; or a compound containing a triarylamine structure and a triphenylsilyl group typified by 9-[4-(carbazol-9-yl)phenyl]-9-[4-(triphenylsilyl)phenyl]-9H-fluorene, etc. These materials may also serve as a material for the hole transport layer. These materials may each be formed into a film singly, or a plurality of types of these materials may be mixed and formed into a film, and each may be used as a single layer. It is possible to form a laminate structure constituted by layers each formed singly by the respective materials, or a laminate structure constituted by layers formed by mixing, or a laminate structure constituted by layers each formed singly by the respective materials and layers formed by mixing a plurality of types of materials. These materials can form thin films by known methods, such as vapor deposition, spin coating, ink-jetting, etc.

For the material for the light-emitting layer in the organic EL device of the present invention, it is possible to use, other than the arylamine compound of the present invention, one of various metal complexes such as a metal complex of a quinolinol derivative, e.g., Alq₃, an anthracene derivative, a bisstyrylbenzene derivative, a pyrene derivative, an oxazole derivative, a poly(para-phenylene vinylene) derivative, etc. The light-emitting layer may be constituted by a host material and a dopant material. For the host material, an anthracene derivative may preferably be used, and also, in addition to such light-emitting materials as the arylamine compound of the present invention, it is possible to use, for example, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, etc. For the dopant material, it is possible to use quinacridone, coumarin, rubrene, perylene, a derivative of the above, a benzopyran derivative, a rhodamine derivative, an aminostyryl derivative, etc. These materials may each be formed into a film singly, or a plurality of types of these materials may be mixed and formed into a film, and each may be used as a single layer. It is possible to form a laminate structure constituted by layers each formed singly by the respective materials, or a laminate structure constituted by layers formed by mixing, or a laminate structure constituted by layers each formed singly by the respective materials and layers formed by mixing a plurality of types of materials. These materials can form thin films by known methods, such as vapor deposition, spin coating, ink-jetting, etc.

It is also possible to use a phosphorescent substance for the light-emitting material. For the phosphorescent substance, it is possible to use a phosphorescent substance such as a metal complex of iridium, platinum, etc. Examples may include green phosphorescent substances such as Ir(ppy)₃ etc., blue phosphorescent substances such as FIrpic, FIr6, etc., and red phosphorescent substances such as Btp₂Ir(acac) etc. As regards host materials, examples of the hole inj ecting/transporting host material may include a carbazole derivative such as 4,4'-di(N-carbazolyl)biphenyl (abbreviated hereinbelow as "CBP"), TCTA, mCP, etc., as well as the arylamine compound of the present invention. Examples of the electron-transporting host material may include p-bis(triphenylsilyl)benzene (abbreviated hereinbelow as "UGH2"), 2,2',2"-(1,3,5-phenylene)-tris(1-phenyl-1H-benzimidazole) (abbreviated hereinbelow as "TPBI"), etc. By using such materials, it is possible to produce high-performance organic EL devices.

To avoid concentration quenching, doping of the host material(s) with a phosphorescent substance is preferably performed by co-vapor deposition within a range of 1 to 30 wt.% with respect to the entire light-emitting layer.

Further, for the light-emitting material, it is possible to use a material emitting delayed fluorescence, e.g., PIC-TRZ, CC2TA, PXZ-TRZ, a CDCB derivative such as 4CzIPN, etc. (see, for example, Non-Patent Literature 3). These materials can form thin films by known methods, such as vapor deposition, spin coating, ink-jetting, etc.

For the material for the hole blocking layer in the organic EL device of the present invention, it is possible to use a compound having a hole blocking action, with examples including phenanthroline derivatives such as bathocuproine (abbreviated hereinbelow as "BCP"), metal complexes of a quinolinol derivative such as BAlq, various rare-earth complexes, oxazole derivatives, triazole derivatives, triazine derivatives, etc. These materials may also serve as materials for the electron transport layer. These materials may each be formed into a film singly, or a plurality of types of these materials may be mixed and formed into a film, and each may be used as a single layer. It is possible to form a laminate structure constituted by layers each formed singly by the respective materials, or a laminate structure constituted by layers formed by mixing, or a laminate structure constituted by layers each formed singly by the respective materials and layers formed by mixing a plurality of types of materials. These materials can form thin films by known methods, such as vapor deposition, spin coating, ink-jetting, etc.

For the material for the electron transport layer in the organic EL device of the present invention, it is possible to use a metal complex of a quinolinol derivative such as Alq₃, BAlq, etc., one of various metal complexes, a triazole derivative, a triazine derivative, an oxadiazole derivative, a pyridine derivative, a pyrimidine derivative, a benzimidazole derivative, a thiadiazole derivative, an anthracene derivative, a carbodiimide derivative, a quinoxaline derivative, a pyridoindole derivative, a phenanthroline derivative, a silole derivative, etc. These materials may each be formed into a film singly, or a plurality of types of these materials may be mixed and formed into a film, and each may be used as a single layer. It is possible to form a laminate structure constituted by layers each formed singly by the respective materials, or a laminate structure constituted by layers formed by mixing, or a laminate structure constituted by layers each formed singly by the respective materials and layers formed by mixing a plurality of types of materials. These materials can form thin films by known methods, such as vapor deposition, spin coating, ink-jetting, etc.

For the material for the electron injection layer in the organic EL device of the present invention, it is possible to use an alkali metal salt such as lithium fluoride, cesium fluoride, etc., an alkaline-earth metal salt such as magnesium fluoride etc., a metal complex of a quinolinol derivative such as quinolinol lithium etc., a metal oxide such as aluminum oxide etc., or a metal such as ytterbium (Yb), samarium (Sm), calcium (Ca), strontium (Sr), cesium (Cs), etc. The electron injection layer may, however, be omitted by suitable selection of the electron transport layer and the cathode.

For the electron injection layer and the electron transport layer, it is possible to use a material ordinarily used for such layers and n-doped with a metal such as cesium etc.

For the cathode in the organic EL device of the present invention, a metal having a low work function, such as aluminum etc., or an alloy having an even lower work function, such as magnesium silver alloy, magnesium indium alloy, aluminum magnesium alloy, etc., may be used as the electrode material.

### Examples

Embodiments of the present invention will be described in further detail below according to working examples. Examples 2, 3, 9, 10, 11, 33, 34, 40, 41, 42 are examples according to the present invention. The rest of the examples are reference examples not forming part of the present invention.

### Example 1:

### Synthesis of bis(biphenyl-4-yl)-(3',5'-diphenyl-1,1':2,' 1"-terphenyl-3"-yl)-amine (Compound (58)):

A reaction vessel was charged with 7.0 g of 2,4,6-triphenyl-bromobenzene, 12.4 g of bis(biphenyl-4-yl)-[3-(4,4,5,5-tetramethyl-[1,3,2]-dioxaborolan-2-yl)-phenyl]-amine, 0.3 g of [1,1'-bis(diphenylphosphino)ferrocene]palladium(II) dichloride dichloromethane adduct, and 3.6 g of sodium hydrogen carbonate, and the mixture was stirred under reflux overnight in a mixed solvent of THF/H₂O. After the mixture was allowed to cool, ethyl acetate/H₂O was added into the system. Then, the organic layer was taken out by extraction and liquid separation and was concentrated, to obtain a crude product. The obtained crude product was purified by column chromatography (adsorbent: silica gel; eluent: dichloromethane/n-heptane), to obtain 9.5 g of a white powder of bis(biphenyl-4-yl)-(3',5'-diphenyl-1,1' :2,' 1"-terphenyl-3"-yl)-amine (Compound (58)) (yield: 74.5%).

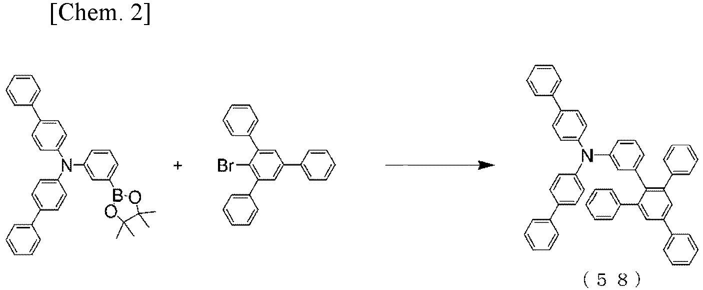

The structure of the obtained white powder was identified by NMR.

The following 39 hydrogen signals were detected with ¹H-NMR (CDCl₃).

δ (ppm) = 7.70 (2H), 7.67 (2H), 7.59 (4H), 7.51-7.29 (19H), 7.24 (4H), 7.00 (1H), 6.87 (6H), 6.67 (1H).

### Example 2:

### Synthesis of (biphenyl-4-yl)-(3',5'-diphenyl-1,1':2',1"-terphenyl-3"-yl)-(4-naphthalen-1-yl-phenyl)-amine (Compound (59)):

A reaction vessel was charged with 10.0 g of 2,4,6-triphenyl-bromobenzene, 22.3 g of (biphenyl-4-yl)-(4-naphthalen-1-yl-phenyl)-[3-(4,4,5,5-tetramethyl-[1,3,2]-dioxaborolan-2-yl)-phenyl]-amine, 0.4 g of [1,1'-bis(diphenylphosphino)ferrocene]palladium(II) dichloride dichloromethane adduct, and 5.1 g of sodium hydrogen carbonate, and the mixture was stirred under reflux overnight in a mixed solvent of THF/H₂O. After the mixture was allowed to cool, ethyl acetate/H₂O was added into the system. Then, the organic layer was taken out by extraction and liquid separation and was concentrated, to obtain a crude product. The obtained crude product was purified by column chromatography (adsorbent: silica gel; eluent: dichloromethane/n-heptane), to obtain 16.0 g of a white powder of (biphenyl-4-yl)-(3',5'-diphenyl-1,1':2',1"-terphenyl-3"-yl)-(4-naphthalen-1-yl-phenyl)-amine (Compound (59)) (yield: 82.0%).

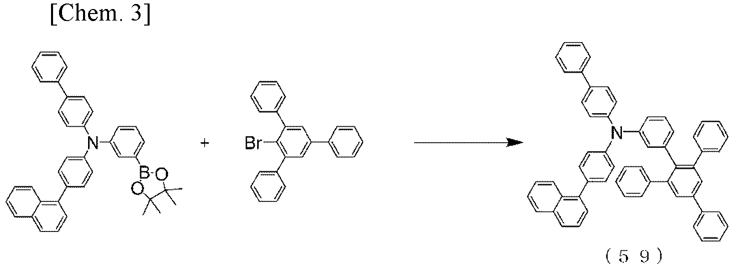

The structure of the obtained white powder was identified by NMR.

The following 41 hydrogen signals were detected with ¹H-NMR (CDCl₃).

δ (ppm) = 8.00 (2H), 7.87 (1H), 7.71 (2H), 7.69 (2H), 7.61 (2H), 7.59-7.43 (10H), 7.42-7.22 (14H), 7.03 (1H), 6.98-6.86 (6H), 6.69 (1H).

### Example 3:

### Synthesis of (biphenyl-4-yl)-(3',5'-diphenyl-1,1':2',1"-terphenyl-3"-yl)-(4-naphthalen-2-yl-phenyl)-amine (Compound (60)):

A reaction vessel was charged with 11.0 g of 2,4,6-triphenyl-bromobenzene, 24.6 g of (biphenyl-4-yl)-(4-naphthalen-2-yl-phenyl)-[3-(4,4,5,5-tetramethyl-[1,3,2]-dioxaborolan-2-yl)-phenyl]-amine, 0.5 g of [1,1'-bis(diphenylphosphino)ferrocene]palladium(II) dichloride dichloromethane adduct, and 5.6 g of sodium hydrogen carbonate, and the mixture was stirred under reflux overnight in a mixed solvent of THF/H₂O. After the mixture was allowed to cool, ethyl acetate/H₂O was added into the system. Then, the organic layer was taken out by extraction and liquid separation and was concentrated, to obtain a crude product. The obtained crude product was purified by column chromatography (adsorbent: silica gel; eluent: dichloromethane/n-heptane), to obtain 15.5 g of a white powder of (biphenyl-4-yl)-(3',5'-diphenyl-1,1':2',1"-terphenyl-3"-yl)-(4-naphthalen-2-yl-phenyl)-amine (Compound (60)) (yield: 72.0%).

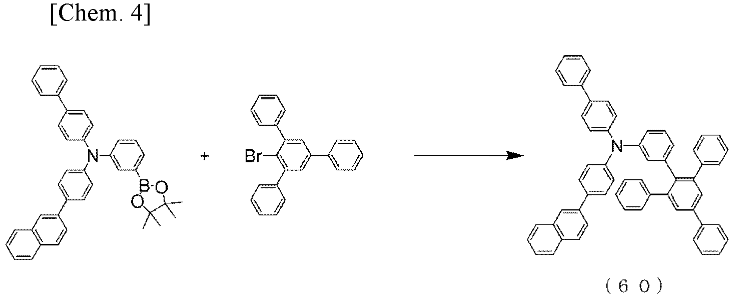

The structure of the obtained white powder was identified by NMR.

The following 41 hydrogen signals were detected with ¹H-NMR (CDCl₃).

δ (ppm) = 8.00 (1H), 7.91 (1H), 7.89 (2H), 7.73 (1H), 7.67 (4H), 7.60-7.37 (12H), 7.37-7.27 (8H), 7.25-7.19 (4H), 6.99 (1H), 6.89 (2H), 6.85 (4H), 6.65 (1H).

### Example 4:

### Synthesis of (3',5'-diphenyl-1,1':2',1"-terphenyl-3"-yl)-(4-phenanthren-9-yl-phenyl)-phenyl-amine (Compound (15)):

A reaction vessel was charged with 13.7 g of (3',5'-diphenyl-1,1':2',1"-terphenyl-3"-yl)-(4-phenanthren-9-yl-phenyl)-amine, 4.0 g of bromobenzene, 0.1 g of palladium(II) acetate, 0.1 g of tri-t-butylphosphine, and 4.1 g of sodium t-butoxide, and the mixture was stirred under reflux for 6 hours in a toluene solvent. After the mixture was allowed to cool, a filtrate obtained by filtering was concentrated, to obtain a crude product. The obtained crude product was purified by crystallization with a dichloromethane/acetone mixed solvent, to obtain 6.7 g of a pale-yellow powder of (3',5'-diphenyl-1,1' :2',1"-terphenyl-3"-yl)-(4-phenanthren-9-yl-phenyl)-phenyl-amine (Compound (15)) (yield: 43.8%).

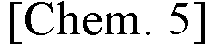

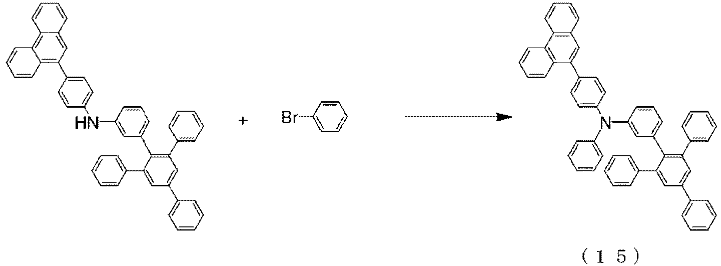

The structure of the obtained pale-yellow powder was identified by NMR.

The following 39 hydrogen signals were detected with ¹H-NMR (CDCl₃).

δ (ppm) = 8.79 (1H), 8.72 (1H), 8.02 (1H), 7.89 (1H), 7.72-7.56 (9H), 7.45 (2H), 7.37 (1H), 7.33-7.17 (14H), 6.98 (2H), 6.89 (3H), 6.83 (3H), 6.64 (1H).

### Example 5:

### Synthesis of (3',5'-diphenyl-1,1':2',1"-terphenyl-3"-yl)-phenyl-(1,1':4',1"-terphenyl-4-yl)-amine (Compound (31)):

A reaction vessel was charged with 10.0 g of (3',5'-diphenyl-1,1':2',1"-terphenyl-3"-yl)-phenyl-amine, 7.8 g of 4-bromo-[1,1' :4',1"]terphenyl, 0.1 g of palladium(II) acetate, 0.2 g of tri-t-butylphosphine, and 3.0 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 8.0 g of a white powder of (3',5'-diphenyl-1,1':2',1"-terphenyl-3"-yl)-phenyl-(1,1':4',1"-terphenyl-4-yl)-amine (Compound (31)) (yield: 54.0%).

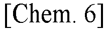

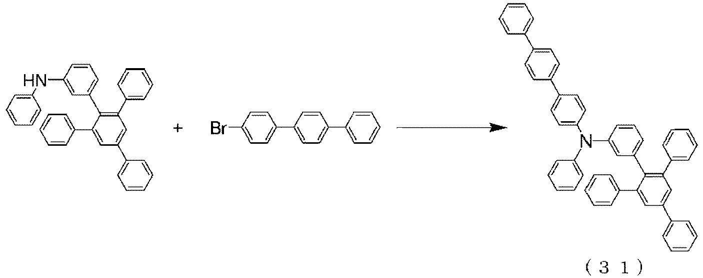

The structure of the obtained white powder was identified by NMR.

The following 39 hydrogen signals were detected with ¹H-NMR (CDCl₃).

δ (ppm) = 7.69-7.60 (10H), 7.45 (4H), 7.40 (2H), 7.36 (2H), 7.29 (6H), 7.17 (2H), 7.20 (4H), 6.95 (2H), 6.80 (5H), 6.77 (1H), 6.62 (1H).

### Example 6:

### Synthesis of (3',5'-diphenyl-1,1':2',1"-terphenyl-3"-yl)-(4'-naphthalen-1-yl-biphenyl-4-yl)-phenyl-amine (Compound (32)):

A reaction vessel was charged with 12.0 g of (3',5'-diphenyl-1,1':2',1"-terphenyl-3"-yl)-phenyl-amine, 10.0 g of 1-(4'-bromo-biphenyl-4-yl)-naphthalene, 0.1 g of palladium(II) acetate, 0.2 g of tri-t-butylphosphine, and 2.9 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 16.4 g of a white powder of (3',5'-diphenyl-1,1':2',1"-terphenyl-3"-yl)-phenyl-(4'-naphthalen-1-yl-biphenyl-4-yl)-amine (Compound (32)) (yield: 86.0%).

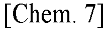

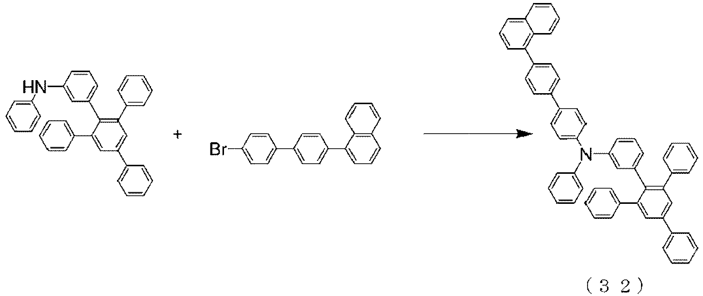

The structure of the obtained white powder was identified by NMR.

The following 41 hydrogen signals were detected with ¹H-NMR (CDCl₃).

δ (ppm) = 8.00 (1H), 7.92 (1H), 7.87 (1H), 7.68 (4H), 7.65 (2H), 7.59-7.41 (10H), 7.36 (1H), 7.30 (2H), 7.29 (4H), 7.21 (4H), 7.18 (2H), 6.96 (2H), 6.82 (5H), 6.79 (1H), 6.63 (1H).

### Example 7:

### Synthesis of (3',5'-diphenyl-1,1':2',1"-terphenyl-3"-yl)-(4'-naphthalen-2-yl-biphenyl-4-yl)-phenyl-amine (Compound (33)):

A reaction vessel was charged with 12.0 g of (3',5'-diphenyl-1,1':2',1"-terphenyl-3"-yl)-phenyl-amine, 10.0 g of 2-(4'-bromo-biphenyl-4-yl)-naphthalene, 0.1 g of palladium(II) acetate, 0.2 g of tri-t-butylphosphine, and 2.9 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a chlorobenzene/acetone mixed solvent, to obtain 14.9 g of a white powder of (3',5'-diphenyl-1,1':2',1"-terphenyl-3"-yl)-phenyl-(4'-naphthalen-2-yl-biphenyl-4-yl)-amine (Compound (33)) (yield: 78.4%).

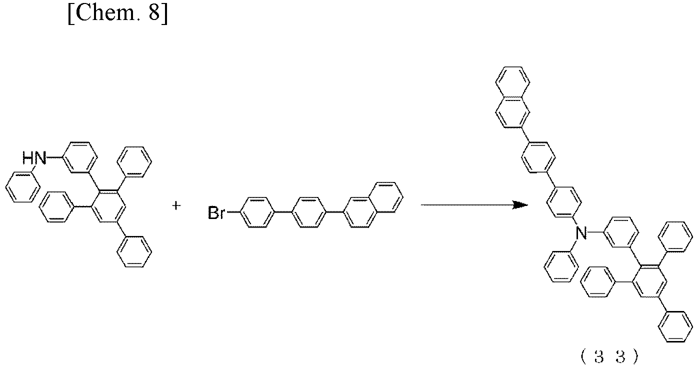

The structure of the obtained white powder was identified by NMR.

The following 41 hydrogen signals were detected with ¹H-NMR (CDCl₃).

δ (ppm) = 8.09 (1H), 7.92 (2H), 7.87 (1H), 7.80 (3H), 7.68 (4H), 7.67 (2H), 7.50 (2H), 7.45 (1H), 7.43 (3H), 7.36 (1H), 7.32-7.26 (6H), 7.21 (4H), 7.18 (2H), 6.97 (1H), 6.96 (1H), 6.85-6.75 (6H), 6.62 (1H).

### Example 8:

### Synthesis of (biphenyl-4-yl)-(3',5'-diphenyl-1,1':2',1"-terphenyl-3"-yl)-(phenanthren-9-yl)-amine (Compound (61)):

A reaction vessel was charged with 10.0 g of 2,4,6-triphenyl-bromobenzene, 18.5 g of (biphenyl-4-yl)-(phenanthren-9-yl)-[3-(4,4,5,5-tetramethyl-[1,3,2]-dioxaborolan-2-yl)-phenyl]-amine, 0.4 g of [1,1'-bis(diphenylphosphino)ferrocene]palladium(II) dichloride dichloromethane adduct, and 5.1 g of sodium hydrogen carbonate, and the mixture was stirred under reflux overnight in a mixed solvent of THF/H₂O. After the mixture was allowed to cool, ethyl acetate/H₂O was added into the system. Then, the organic layer was taken out by extraction and liquid separation and was concentrated, to obtain a crude product. The obtained crude product was purified by column chromatography (adsorbent: silica gel; eluent: dichloromethane/n-heptane), to obtain 7.0 g of a pale-yellow powder of (biphenyl-4-yl)-(3',5'-diphenyl-1,1':2',1"-terphenyl-3"-yl)-(phenanthren-9-yl)-amine (Compound (61)) (yield: 37.0%).

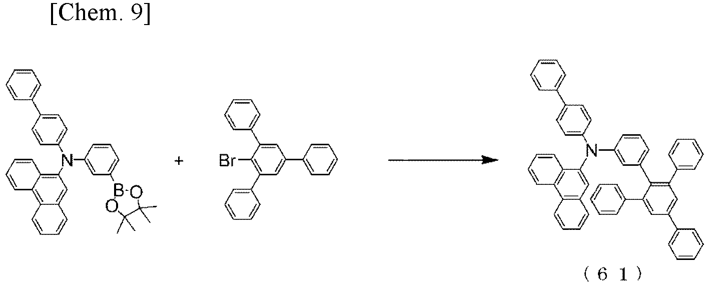

The structure of the obtained pale-yellow powder was identified by NMR.

The following 39 hydrogen signals were detected with ¹H-NMR (CDCl₃).

δ (ppm) = 8.72 (1H), 8.69 (1H), 7.90 (1H), 7.74 (1H), 7.65 (4H), 7.64 (2H), 7.58 (1H), 7.51 (3H), 7.48-7.32 (6H), 7.31-7.22 (9H), 7.19 (4H), 6.99 (1H), 6.82 (1H), 6.62 (3H), 6.52 (1H).

### Example 9:

### Synthesis of (3',5'-diphenyl-1,1':2',1"-terphenyl-3"-yl)-(4-naphthalen-1-yl-phenyl)-(4-naphthalen-2-yl-phenyl)-amine (Compound (76)):

A reaction vessel was charged with 13.4 g of (4-naphthalen-2-yl-phenyl)-(3',5'-diphenyl-1,1':2',1"-terphenyl-3"-yl)-amine, 7.6 g of 1-(4-bromo-phenyl)-naphthalene, 0.1 g of palladium(II) acetate, 0.1 g of tri-t-butylphosphine, and 4.3 g of sodium t-butoxide, and the mixture was stirred under reflux for 6 hours in a toluene solvent. After the mixture was allowed to cool, a filtrate obtained by filtering was concentrated, to obtain a crude product. The obtained crude product was purified by crystallization with a dichloromethane/acetone mixed solvent, to obtain 10.6 g of a white powder of (3',5'-diphenyl-1,1':2',1"-terphenyl-3"-yl)-(4-naphthalen-1-yl-phenyl)-(4-naphthalen-2-yl-phenyl)-amine (Compound (76)) (yield: 59.2%).

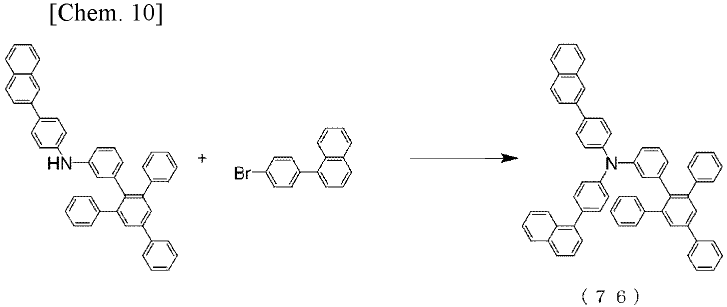

The structure of the obtained white powder was identified by NMR.

The following 43 hydrogen signals were detected with ¹H-NMR (CDCl₃).

δ (ppm) = 8.02 (2H), 7.91 (3H), 7.86 (2H), 7.75 (1H), 7.69 (2H), 7.67 (2H), 7.57 (2H), 7.54-7.41 (8H), 7.37 (1H), 7.33-7.22 (12H), 7.02 (1H), 6.97 (2H), 6.94 (1H), 6.90 (1H), 6.88 (2H), 6.67 (1H).

### Example 10:

### Synthesis of bis(4-naphthalen-2-yl-phenyl)-(3',5'-diphenyl-1,1':2',1"-terphenyl-3"-yl)-amine (Compound (77)):

A reaction vessel was charged with 7.0 g of (3',5'-diphenyl-1,1' :2',1"-terphenyl-3"-yl)-amine, 11.0 g of 2-(4-bromo-phenyl)-naphthalene, 0.6 g of tris(dibenzylideneacetone)dipalladium(0), 4.4 g of 2,2'-bis(diphenylphosphino)-1,1'-binaphthyl, and 6.8 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a dichloromethane/acetone mixed solvent, to obtain 4.1 g of a white powder of bis(4-naphthalen-2-yl-phenyl)-(3',5'-diphenyl-1,1':2',1"-terphenyl-3"-yl)-amine (Compound (77)) (yield: 29.1%).

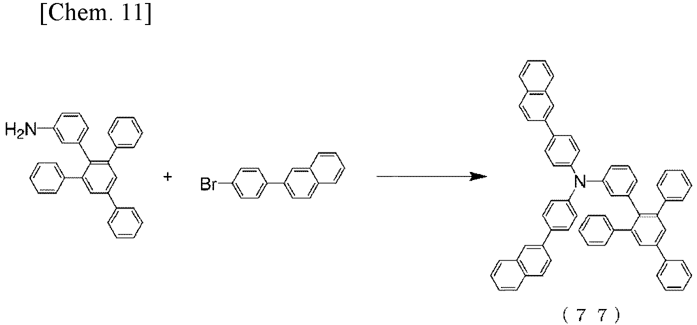

The structure of the obtained white powder was identified by NMR.

The following 43 hydrogen signals were detected with ¹H-NMR (CDCl₃).

δ (ppm) = 8.01 (2H), 7.90 (4H), 7.86 (2H), 7.74 (2H), 7.68 (2H), 7.66 (2H), 7.54 (4H), 7.49 (4H), 7.43 (2H), 7.36 (2H), 7.32 (5H), 7.23 (4H), 7.00 (1H), 6.91 (3H), 6.88 (3H), 6.67 (1H).

### Example 11:

### Synthesis of bis(4-naphthalen-1-yl-phenyl)-(3',5'-diphenyl-1,1':2',1"-terphenyl-3"-yl)-amine (Compound (78)):

A reaction vessel was charged with 7.0 g of (3',5'-diphenyl-1,1' :2',1"-terphenyl-3"-yl)-amine, 11.0 g of 1-(4-bromo-phenyl)-naphthalene, 0.6 g of tris(dibenzylideneacetone)dipalladium(0), 4.4 g of 2,2'-bis(diphenylphosphino)-1,1'-binaphthyl, and 6.8 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a dichloromethane/acetone mixed solvent, to obtain 7.7 g of a white powder of bis(4-naphthalen-1-yl-phenyl)-(3',5'-diphenyl-1,1':2',1"-terphenyl-3"-yl)-amine (Compound (78)) (yield: 54.6%).

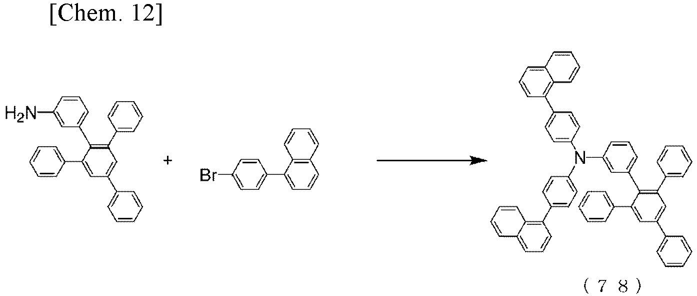

The structure of the obtained white powder was identified by NMR.

The following 43 hydrogen signals were detected with ¹H-NMR (CDCl₃).

δ (ppm) = 8.06 (1H), 7.94 (1H), 7.88 (1H), 7.79 (2H), 7.73 (4H), 7.62 (2H), 7.58-7.47 (5H), 7.44 (2H), 7.37 (2H), 7.32-7.19 (22H), 7.12 (1H).

### Example 12:

### Synthesis of (3',5'-diphenyl-1,1':2',1"-terphenyl-3"-yl)-(4-naphthalen-2-yl-phenyl)-(phenanthren-9-yl)-amine (Compound (79)):

A reaction vessel was charged with 13.4 g of (4-naphthalen-2-yl-phenyl)-(3',5'-diphenyl-1,1':2',1"-terphenyl-3"-yl)-amine, 6.9 g of 9-bromo-phenanthrene, 0.1 g of palladium(II) acetate, 0.1 g of tri-t-butylphosphine, and 4.3 g of sodium t-butoxide, and the mixture was stirred under reflux for 6 hours in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 10.8 g of a pale-yellow powder of (3',5'-diphenyl-1,1':2',1"-terphenyl-3"-yl)-(4-naphthalen-2-yl-phenyl)-(phenanthren-9-yl)-amine (Compound (79)) (yield: 62.4%).

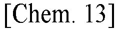

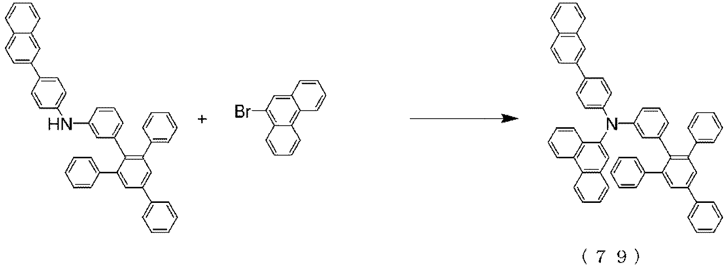

The structure of the obtained pale-yellow powder was identified by NMR.

The following 41 hydrogen signals were detected with ¹H-NMR (CDCl₃).

δ (ppm) = 8.74 (1H), 8.70 (1H), 7.95 (1H), 7.92 (1H), 7.88 (1H), 7.86 (1H), 7.84 (1H), 7.75 (1H), 7.69 (1H), 7.66 (6H), 7.59 (1H), 7.49 (2H), 7.46 (1H), 7.43 (4H), 7.40 (1H), 7.35 (1H), 7.27 (6H), 7.20 (4H), 7.01 (1H), 6.84 (1H), 6.67 (2H), 6.65 (1H), 6.54 (1H).

### Example 13:

### Synthesis of (biphenyl-4-yl)-phenyl-(3',5'-diphenyl-1,1':2',1":3"-1‴-quaterphenyl-4"'-yl)-amine (Compound (96)):

A reaction vessel was charged with 7.5 g of 2,4,6-triphenyl-bromobenzene, 12.2 g of (biphenyl-4-yl)-phenyl-[3'-(4,4,5,5-tetramethyl-[1,3,2]-dioxaborolan-2-yl)-biphenyl-4-yl]-amine, 0.5 g of tetrakis(triphenylphosphine)palladium(0), and 4.0 g of potassium carbonate, and the mixture was stirred under reflux overnight in a mixed solvent of toluene/EtOH/H₂O. After the mixture was allowed to cool, ethyl acetate/H₂O was added into the system. Then, the organic layer was taken out by extraction and liquid separation and was concentrated, to obtain a crude product. The obtained crude product was purified by column chromatography (adsorbent: silica gel; eluent: toluene/n-heptane), to obtain 12.9 g of a white powder of (biphenyl-4-yl)-phenyl-(3',5'-diphenyl-1,1':2',1":3"-1‴-quaterphenyl-4"'-yl)-amine (Compound (96)) (yield: 94.4%).

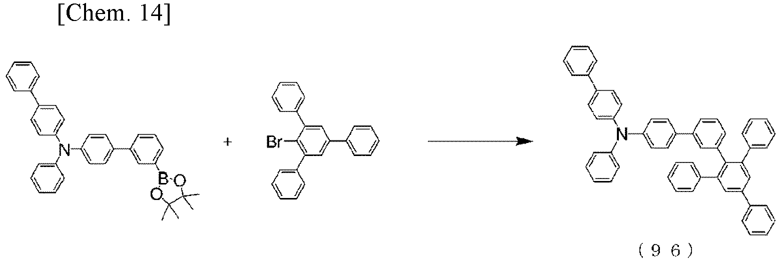

The structure of the obtained white powder was identified by NMR.

The following 39 hydrogen signals were detected with ¹H-NMR (CDCl₃).

δ (ppm) = 7.72 (2H), 7.71 (2H), 7.58 (2H), 7.47 (4H), 7.42 (2H), 7.37 (1H), 7.30 (2H), 7.27 (1H), 7.24-7.16 (11H), 7.14 (4H), 7.10-6.98 (7H), 6.80 (1H).

### Example 14:

### Synthesis of (3',5'-diphenyl-1,1':2',1":3"-1‴-quaterphenyl-4"'-yl)-(4-naphthalen-1-yl-phenyl)-phenyl-amine (Compound (100)):

A reaction vessel was charged with 10.0 g of (3',5'-diphenyl-1,1':2',1":3"-1‴-quaterphenyl-4‴-yl)-phenyl-amine, 5.7 g of 1-(4-bromo-phenyl)-naphthalene, 0.1 g of palladium(II) acetate, 0.2 g of tri-t-butylphosphine, and 2.1 g of sodium t-butoxide, and the mixture was stirred under reflux overnight in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 8.3 g of a white powder of (3',5'-diphenyl-1,1':2',1":3"-1‴-quaterphenyl-4‴-yl)-(4-naphthalen-1-yl-phenyl)-phenyl-amine (Compound (100)) (yield: 60.7%).

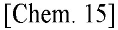

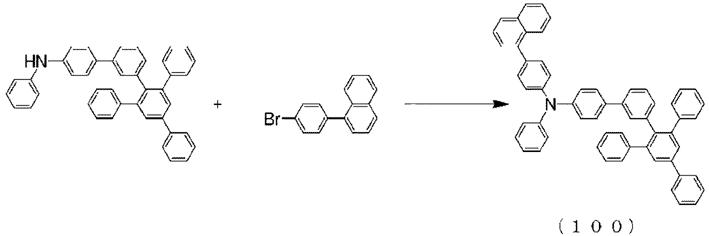

The structure of the obtained white powder was identified by NMR.

The following 41 hydrogen signals were detected with ¹H-NMR (CDCl₃).

δ (ppm) = 8.02 (1H), 7.90 (1H), 7.84 (1H), 7.72 (2H), 7.71 (2H), 7.51 (2H), 7.46 (4H), 7.38 (3H), 7.31 (2H), 7.24-7.01 (22H), 6.80 (1H).

### Example 15:

### Synthesis of (3',5'-diphenyl-1,1':2',1":3"-1‴-quaterphenyl-4‴-yl)-(4-naphthalen-2-yl-phenyl)-phenyl-amine (Compound (101)):

A reaction vessel was charged with 10.0 g of (3',5'-diphenyl-1,1':2',1":3"-1‴-quaterphenyl-4‴-yl)-phenyl-amine, 6.2 g of 2-(4-bromo-phenyl)-naphthalene, 0.1 g of palladium(II) acetate, 0.2 g of tri-t-butylphosphine, and 2.1 g of sodium t-butoxide, and the mixture was stirred under reflux overnight in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 7.7 g of a white powder of (3',5'-diphenyl-1,1':2',1":3"-1‴-quaterphenyl-4‴-yl)-(4-naphthalen-2-yl-phenyl)-phenyl-amine (Compound (101)) (yield: 56.3%).

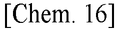

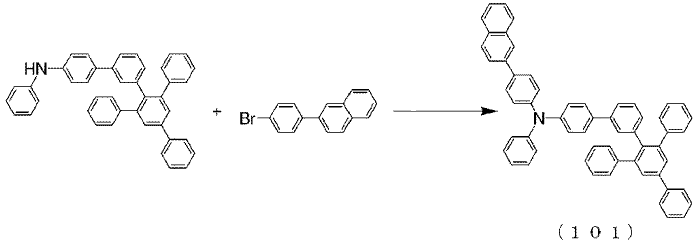

The structure of the obtained white powder was identified by NMR.

The following 41 hydrogen signals were detected with ¹H-NMR (CDCl₃).

δ (ppm) = 8.02 (1H), 7.89 (2H), 7.85 (1H), 7.73 (2H), 7.71 (3H), 7.61 (2H), 7.47 (4H), 7.37 (1H), 7.29 (2H), 7.25-7.13 (15H), 7.12-6.99 (7H), 6.80 (1H).

### Example 16:

### Synthesis of (3',5'-diphenyl-1,1':2',1":3"-1‴-quaterphenyl-4‴-yl)-(4-phenanthren-9-yl-phenyl)-phenyl-amine (Compound (102)):

A reaction vessel was charged with 11.0 g of (3',5'-diphenyl-1,1':2',1":3"-1‴-quaterphenyl-4‴-yl)-phenyl-amine, 8.0 g of 9-(4-bromo-phenyl)-phenanthrene, 0.1 g of palladium(II) acetate, 0.2 g of tri-t-butylphosphine, and 2.3 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 12.6 g of a white powder of (3',5'-diphenyl-1,1':2',1":3"-1‴-quaterphenyl-4‴-yl)-(4-phenanthren-9-yl-phenyl)-phenyl-amine (Compound (102)) (yield: 78.5%).

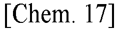

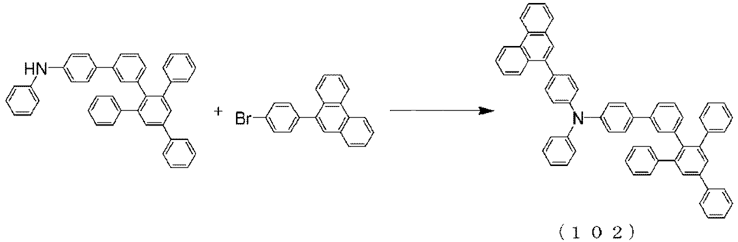

The structure of the obtained white powder was identified by NMR.

The following 43 hydrogen signals were detected with ¹H-NMR (CDCl₃).

δ (ppm) = 8.78 (1H), 8.72 (1H), 8.06 (1H), 7.90 (1H), 7.73 (2H), 7.71 (3H), 7.67 (2H), 7.62 (1H), 7.58 (1H), 7.46 (2H), 7.43 (2H), 7.37 (1H), 7.32 (2H), 7.24-7.17 (15H), 7.17-7.02 (7H), 6.81 (1H).

### Example 17:

### Synthesis of (3',5'-diphenyl-1,1":2',1":3"-1‴-quaterphenyl-4‴-yl)-phenyl-(1,1':4',1"-terphenyl-4-yl)-amine (Compound (107)):

A reaction vessel was charged with 10.0 g of (3',5'-diphenyl-1,1':2',1":3"-1‴-quaterphenyl-4‴-yl)-phenyl-amine, 6.7 g of 4-bromo-[1,1':4',1"]terphenyl, 0.1 g of palladium(II) acetate, 0.2 g of tri-t-butylphosphine, and 2.1 g of sodium t-butoxide, and the mixture was stirred under reflux overnight in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 9.4 g of a pale-yellow powder of (3',5'-diphenyl-l, 1':2',1":3"-1‴-quaterphenyl-4‴-yl)-phenyl-(1,1':4',1"-terphenyl-4-yl)-amine (Compound (107)) (yield: 66.4%).

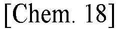

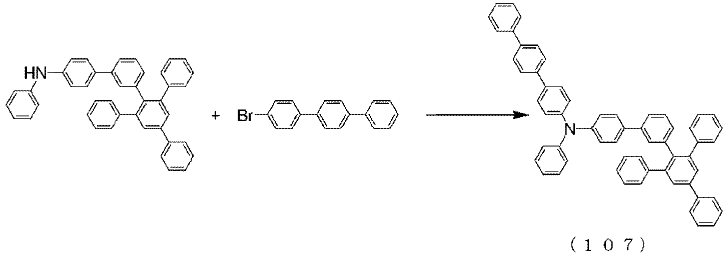

The structure of the obtained pale-yellow powder was identified by NMR.

The following 43 hydrogen signals were detected with ¹H-NMR (CDCl₃).

δ (ppm) = 7.72 (2H), 7.71 (2H), 7.66 (4H), 7.64 (2H), 7.53 (2H), 7.46 (4H), 7.36 (2H), 7.28 (2H), 7.24-7.12 (15H), 7.11-6.99 (7H), 6.80 (1H).

### Example 18:

### Synthesis of bis(biphenyl-4-yl)-(3',5'-diphenyl-1,1':2',1":3"-1‴-quaterphenyl-4"'-yl)-amine (Compound (115)):

A reaction vessel was charged with 10.0 g of biphenyl-4-yl-(3',5'-diphenyl-1,1':2',1":3"-1‴-quaterphenyl-4‴-yl)-amine, 4.5 g of 4-bromo-biphenyl, 0.7 g of tris(dibenzylideneacetone)dipalladium(0), 0.7 g of tri-t-butylphosphine, and 2.0 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 10.5 g of a white powder of bis(biphenyl-4-yl)-(3',5'-diphenyl-1,1':2',1":3"-1‴-quaterphenyl-4‴-yl)-amine (Compound (115)) (yield: 84.5%).

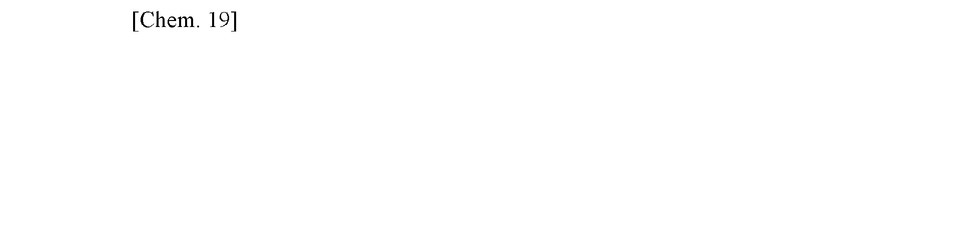

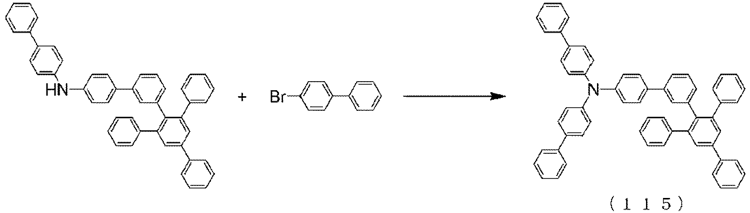

The structure of the obtained white powder was identified by NMR.

The following 43 hydrogen signals were detected with ¹H-NMR (CDCl₃).

δ (ppm) = 7.73 (2H), 7.72 (2H), 7.59 (4H), 7.51 (4H), 7.47 (2H), 7.43 (4H), 7.38 (1H), 7.32 (2H), 7.24-7.15 (15H), 7.14-7.01 (6H), 6.81 (1H).

### Example 19:

### Synthesis of (biphenyl-4-yl)-(3',5'-diphenyl-1,1':2',1":3"-1‴-quaterphenyl-4‴-yl)-(4-naphthalen-2-yl-phenyl)-amine (Compound (116)):

A reaction vessel was charged with 10.0 g of biphenyl-4-yl-(3',5'-diphenyl-1,1':2',1":3"-1‴-quaterphenyl-4‴-yl)-amine, 5.4 g of 2-(4-bromo-phenyl)-naphthalene, 0.7 g of tris(dibenzylideneacetone)dipalladium(0), 0.7 g of tri-t-butylphosphine, and 2.0 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 9.1 g of a pale-yellow powder of (biphenyl-4-yl)-(3',5'-diphenyl-1,1':2',1":3"-1‴-quaterphenyl-4‴-yl)-(4-naphthalen-2-yl-phenyl)-amine (Compound (116)) (yield: 68.8%).

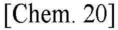

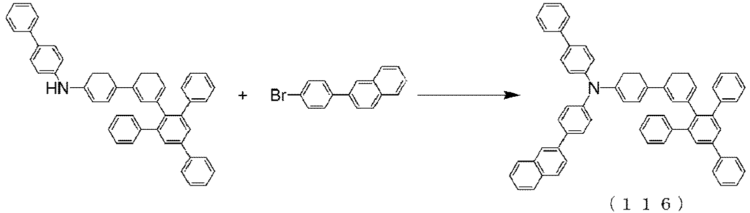

The structure of the obtained pale-yellow powder was identified by NMR.

The following 45 hydrogen signals were detected with ¹H-NMR (CDCl₃).

δ (ppm) = 8.03 (1H), 7.90 (2H), 7.86 (1H), 7.75 (2H), 7.72 (3H), 7.64 (2H), 7.60 (2H), 7.54-7.41 (8H), 7.38 (1H), 7.33 (1H), 7.25-7.17 (15H), 7.16-7.02 (6H), 6.81 (1H).

### Example 20:

### Synthesis of (biphenyl-4-yl)-(3',5'-diphenyl-1,1':2',1":3"-1‴-quaterphenyl-4‴-yl)-(4-naphthalen-1-yl-phenyl)-amine (Compound (117)):

A reaction vessel was charged with 10.0 g of biphenyl-4-yl-(3',5'-diphenyl-1,1':2',1":3"-1‴-quaterphenyl-4‴-yl)-amine, 5.4 g of 1-(4-bromo-phenyl)-naphthalene, 0.7 g of tris(dibenzylideneacetone)dipalladium(0), 0.7 g of tri-t-butylphosphine, and 2.3 g of sodium t-butoxide, and the mixture was stirred under reflux overnight in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 12.5 g of a pale-yellow powder of (biphenyl-4-yl)-(3',5'-diphenyl-1,1':2',1":3"-1‴-quaterphenyl-4‴-yl)-(4-naphthalen-1-yl-phenyl)-amine (Compound (117)) (yield: 94.5%).

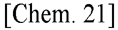

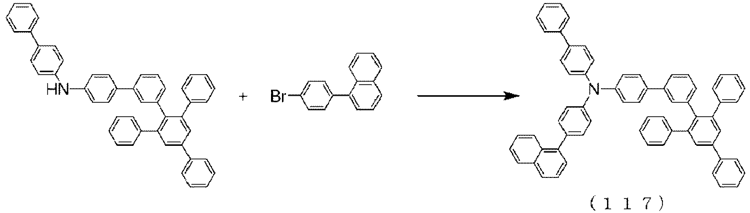

The structure of the obtained pale-yellow powder was identified by NMR.

The following 45 hydrogen signals were detected with ¹H-NMR (CDCl₃).

δ (ppm) = 8.04 (1H), 7.91 (1H), 7.85 (1H), 7.73 (2H), 7.72 (2H), 7.61 (2H), 7.56-7.35 (13H), 7.32 (1H), 7.28-7.15 (17H), 7.11 (1H), 7.08 (1H), 7.07 (2H), 6.81 (1H).

### Example 21:

### Synthesis of (biphenyl-4-yl)-(3',5'-diphenyl-1,1':2',1":3"-1‴-quaterphenyl-4‴-yl)-(phenanthren-2-yl)-amine (Compound (118)):

A reaction vessel was charged with 10.0 g of biphenyl-4-yl-(3',5'-diphenyl-1,1':2',1":3"-1‴-quaterphenyl-4‴-yl)-amine, 4.9 g of 2-bromo-phenanthrene, 0.7 g of tris(dibenzylideneacetone)dipalladium(0), 0.7 g of tri-t-butylphosphine, and 2.3 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 8.7 g of a pale-yellow powder of (biphenyl-4-yl)-(3',5'-diphenyl-1,1':2',1":3"-1‴-quaterphenyl-4‴-yl)-(phenanthren-2-yl)-amine (Compound (118)) (yield: 67.9%).

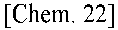

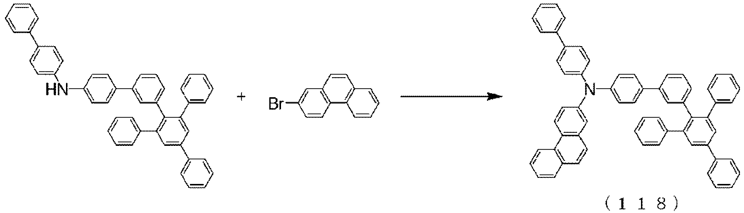

The structure of the obtained pale-yellow powder was identified by NMR.

The following 43 hydrogen signals were detected with ¹H-NMR (CDCl₃).

δ (ppm) = 8.59 (1H), 8.56 (1H), 7.85 (1H), 7.72 (2H), 7.71 (2H), 7.68 (1H), 7.63 (1H), 7.60 (2H), 7.54 (5H), 7.45 (5H), 7.38 (1H), 7.32 (1H), 7.25-7.12 (15H), 7.11 (1H), 7.05 (1H), 7.04 (2H), 6.81 (1H).

### Example 22:

### Synthesis of (biphenyl-4-yl)-(3',5'-diphenyl-1,1':2',1":3"-1‴-quaterphenyl-4‴-yl)-(phenanthren-9-yl)-amine (Compound (119)):

A reaction vessel was charged with 10.0 g of biphenyl-4-yl-(3',5'-diphenyl-1,1':2',1":3"-1‴-quaterphenyl-4‴-yl)-amine, 4.9 g of 9-bromo-phenanthrene, 0.7 g of tris(dibenzylideneacetone)dipalladium(0), 0.7 g of tri-t-butylphosphine, and 2.3 g of sodium t-butoxide, and the mixture was stirred under reflux overnight in a toluene solvent. After the mixture was allowed to cool, a filtrate obtained by filtering was concentrated, to obtain a crude product. The obtained crude product was purified by column chromatography (adsorbent: silica gel; eluent: dichloromethane/n-heptane), to obtain 7.9 g of a pale-yellow powder of (biphenyl-4-yl)-(3',5'-diphenyl-1,1':2',1":3"-1‴-quaterphenyl-4‴-yl)-(phenanthren-9-yl)-amine (Compound (119)) (yield: 61.6%).

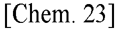

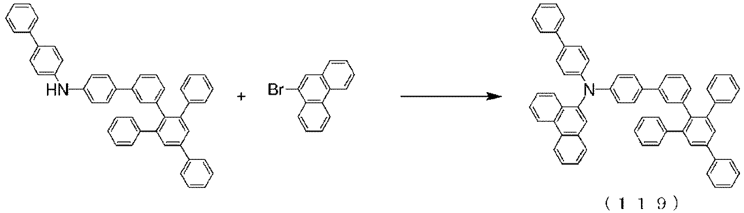

The structure of the obtained pale-yellow powder was identified by NMR.

The following 43 hydrogen signals were detected with ¹H-NMR (CDCl₃).

δ (ppm) = 8.76 (1H), 8.72 (1H), 8.06 (1H), 7.78 (1H), 7.71 (2H), 7.69 (2H), 7.66 (2H), 7.64 (1H), 7.59 (1H), 7.55 (2H), 7.51 (1H), 7.46 (1H), 7.44 (3H), 7.39 (2H), 7.36 (1H), 7.28 (1H), 7.22-7.12 (13H), 7.07 (3H), 7.01 (1H), 6.97 (2H), 6.77 (1H).

### Example 23:

### Synthesis of (3',5'-diphenyl-1,1':2',1"-terphenyl-3"-yl)-(4'-phenanthren-9-yl-biphenyl-4-yl)-phenyl-amine (Compound (127)):

A reaction vessel was charged with 10.0 g of (3',5'-diphenyl-1,1':2',1"-terphenyl-3"-yl)-phenyl-amine, 9.5 g of 9-(4'-bromo-biphenyl-4-yl)-phenanthrene, 0.1 g of palladium(II) acetate, 0.2 g of tri-t-butylphosphine, and 2.4 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 14.6 g of a white powder of (3',5'-diphenyl-1,1':2',1"-terphenyl-3"-yl)-phenyl-(4'-phenanthren-9-yl-biphenyl-4-yl)-amine (Compound (127)) (yield: 86.3%).

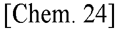

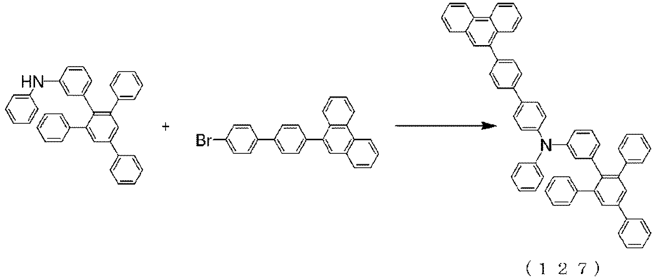

The structure of the obtained white powder was identified by NMR.

The following 43 hydrogen signals were detected with ¹H-NMR (CDCl₃).

δ (ppm) = 8.79 (1H), 8.74 (1H), 8.01 (1H), 7.91 (1H), 7.73 (1H), 7.69 (3H), 7.67 (3H), 7.65 (2H), 7.62 (3H), 7.56 (1H), 7.47 (2H), 7.44 (2H), 7.36 (1H), 7.29 (6H), 7.20 (6H), 6.97 (2H), 6.83 (5H), 6.79 (1H), 6.63 (1H).

### Example 24:

### Synthesis of (biphenyl-4-yl)-(3',5'-diphenyl-1,1':2',1"-terphenyl-3"-yl)-(4-phenanthren-9-yl-phenyl)-amine (Compound (130)):

A reaction vessel was charged with 12.5 g of (3',5'-diphenyl-1,1':2',1"-terphenyl-3"-yl)-(4-phenanthren-9-yl-phenyl)-amine, 5.4 g of 4-bromo-biphenyl, 0.1 g of palladium(II) acetate, 0.1 g of tri-t-butylphosphine, and 3.7 g of sodium t-butoxide, and the mixture was stirred under reflux for 6 hours in a toluene solvent. After the mixture was allowed to cool, a filtrate obtained by filtering was concentrated, to obtain a crude product. The obtained crude product was purified by crystallization with a dichloromethane/acetone mixed solvent, to obtain 9.3 g of a white powder of (biphenyl-4-yl)-(3',5'-diphenyl-1,1':2',1"-terphenyl-3"-yl)-(4-phenanthren-9-yl-phenyl)-amine (Compound (130)) (yield: 60.1%).

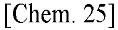

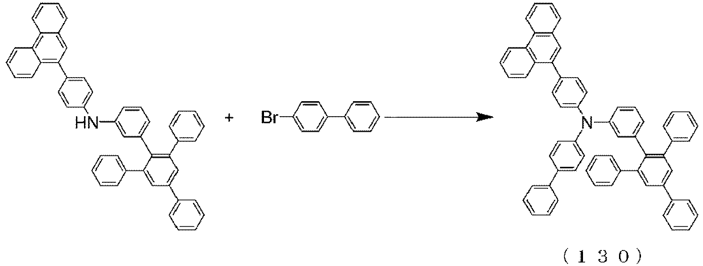

The structure of the obtained white powder was identified by NMR.

The following 43 hydrogen signals were detected with ¹H-NMR (CDCl₃).

δ (ppm) = 8.79 (1H), 8.73 (1H), 8.03 (1H), 7.90 (1H), 7.70 (2H), 7.68 (2H), 7.67 (2H), 7.63 (1H), 7.61 (1H), 7.58 (2H), 7.44 (6H), 7.37 (1H), 7.33 (3H), 7.31-7.21 (11H), 7.01 (1H), 6.94 (3H), 6.89 (3H), 6.67 (1H).

### Example 25:

### Synthesis of (3',5'-diphenyl-1,1':2',1"-terphenyl-3"-yl)-(4-naphthalen-1-yl-phenyl)-(1,1':4',1"-terphenyl-4-yl)-amine (Compound (132)):

A reaction vessel was charged with 8.0 g of (3',5'-diphenyl-1,1' :2',1"-terphenyl-3"-yl)-(1,1':4',1"-terphenyl-4-yl)-amine, 4.3 g of 1-(4-bromo-phenyl)-naphthalene, 0.1 g of palladium(II) acetate, 0.1 g of tri-t-butylphosphine, and 1.6 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 7.9 g of a white powder of (3',5' -diphenyl-1,1':2',1"-terphenyl-3"-yl)-(4-naphthalen-1-yl-phenyl)-(1,1':4',1"-terphenyl-4-yl)-amine (Compound (132)) (yield: 74.5%).

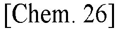

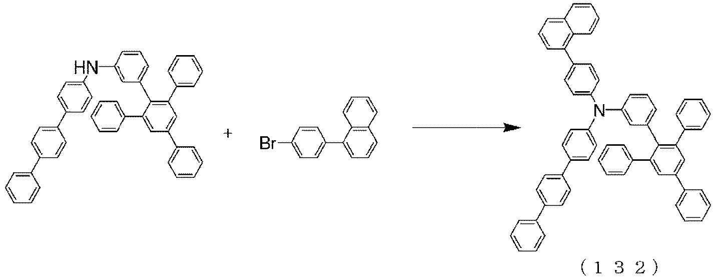

The structure of the obtained white powder was identified by NMR.

The following 45 hydrogen signals were detected with ¹H-NMR (CDCl₃).

δ (ppm) = 8.01 (1H), 7.91 (1H), 7.85 (1H), 7.71-7.62 (10H), 7.56-7.41 (10H), 7.36 (2H), 7.32-7.21 (12H), 7.01 (1H), 6.94 (3H), 6.88 (3H), 6.67 (1H).

### Example 26:

### Synthesis of (3',5'-diphenyl-1,1':2',1"-terphenyl-3"-yl)-(4-naphthalen-2-yl-phenyl)-(1,1':4',1"-terphenyl-4-yl)-amine (Compound (133)):

A reaction vessel was charged with 8.0 g of (3',5'-diphenyl-1,1' :2',1"-terphenyl-3"-yl)-(1,1':4',1"-terphenyl-4-yl)-amine, 4.0 g of 2-(4-bromo-phenyl)-naphthalene, 0.1 g of tris(dibenzylideneacetone)dipalladium(0), 0.1 g of tri-t-butylphosphine, and 1.5 g of sodium t-butoxide, and the mixture was stirred under reflux for 3 hours in a toluene solvent. After the mixture was allowed to cool, a filtrate obtained by filtering was concentrated, to obtain a crude product. The obtained crude product was purified by crystallization with a toluene/acetone mixed solvent, to obtain 8.5 g of a pale-red powder of (3',5'-diphenyl-1,1':2',1"-terphenyl-3"-yl)-(4-naphthalen-2-yl-phenyl)-(1,1':4',1"-terphenyl-4-yl)-amine (Compound (133)) (yield: 80.3%).

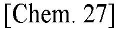

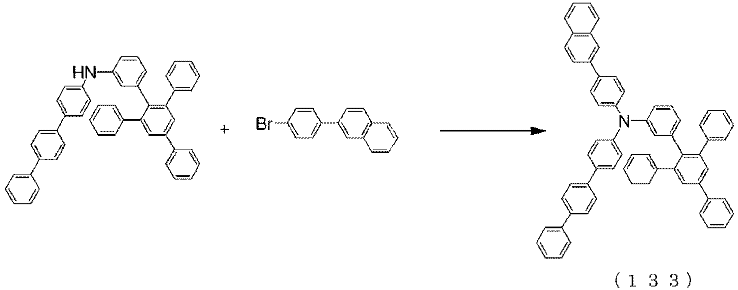

The structure of the obtained pale-red powder was identified by NMR.

The following 45 hydrogen signals were detected with ¹H-NMR (CDCl₃).

δ (ppm) = 8.00 (1H), 7.91 (1H), 7.89 (1H), 7.86 (1H), 7.73 (1H), 7.70-7.62 (10H), 7.53 (2H), 7.51-7.40 (8H), 7.35 (2H), 7.30 (6H), 7.22 (4H), 6.99 (1H), 6.91 (2H), 6.86 (4H), 6.66 (1H).

### Example 27:

### Synthesis of (3',5'-diphenyl-1,1':2',1"-terphenyl-3"-yl)-(3-naphthalen-1-yl-phenyl)-(1,1':4',1"-terphenyl-4-yl)-amine (Compound (134)):

A reaction vessel was charged with 8.0 g of (3',5'-diphenyl-1,1' :2',1"-terphenyl-3"-yl)-(1,1':4',1"-terphenyl-4-yl)-amine, 4.3 g of 1-(3-bromo-phenyl)-naphthalene, 0.1 g of palladium(II) acetate, 0.1 g of tri-t-butylphosphine, and 1.6 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone/n-heptane mixed solvent, to obtain 6.0 g of a white powder of (3',5'-diphenyl-1,1':2',1"-terphenyl-3"-yl)-(3-naphthalen-1-yl-phenyl)-(1,1':4',1"-terphenyl-4-yl)-amine (Compound (134)) (yield: 56.6%).

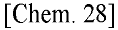

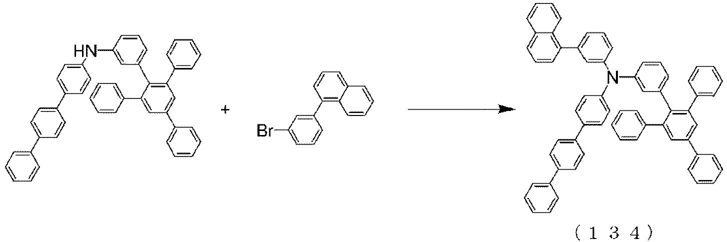

The structure of the obtained white powder was identified by NMR.

The following 45 hydrogen signals were detected with ¹H-NMR (CDCl₃).

δ (ppm) = 7.87 (3H), 7.68-7.59 (10H), 7.50 (1H), 7.48-7.32 (11H), 7.29 (1H), 7.14 (4H), 7.09 (7H), 6.99 (2H), 6.91 (1H), 6.88 (1H), 6.87 (1H), 6.84 (2H), 6.63 (1H).

### Example 28:

### Synthesis of (3',5'-diphenyl-1,1':2',1"-terphenyl-3"-yl)-(3-naphthalen-2-yl-phenyl)-(1,1':4',1"-terphenyl-4-yl)-amine (Compound (135)):

A reaction vessel was charged with 8.0 g of (3',5'-diphenyl-1,1' :2',1"-terphenyl-3"-yl)-(1,1':4',1"-terphenyl-4-yl)-amine, 4.3 g of 2-(3-bromo-phenyl)-naphthalene, 0.1 g of palladium(II) acetate, 0.1 g of tri-t-butylphosphine, and 1.6 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 6.5 g of a white powder of (3',5' -diphenyl-1,1':2',1"-terphenyl-3"-yl)-(3-naphthalen-2-yl-phenyl)-(1,1':4',1"-terphenyl-4-yl)-amine (Compound (135)) (yield: 61.4%).

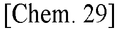

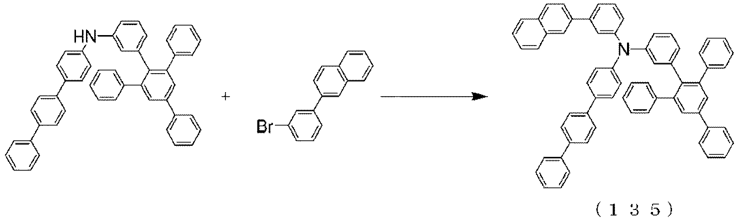

The structure of the obtained white powder was identified by NMR.

The following 45 hydrogen signals were detected with ¹H-NMR (CDCl₃).

δ (ppm) = 7.93 (1H), 7.87 (2H), 7.85 (1H), 7.70-7.61 (11H), 7.53-7.39 (8H), 7.35 (2H), 7.31 (2H), 7.28 (1H), 7.25-7.15 (10H), 6.99 (1H), 6.87 (2H), 6.83 (2H), 6.81 (1H), 6.65 (1H).

### Example 29:

### Synthesis of (3',5'-diphenyl-1,1':2',1"-terphenyl-3"-yl)-(2-naphthalen-1-yl-phenyl)-(1,1':4',1"-terphenyl-4-yl)-amine (Compound (136)):

A reaction vessel was charged with 8.0 g of (3',5'-diphenyl-1,1' :2',1"-terphenyl-3"-yl)-(1,1':4',1"-terphenyl-4-yl)-amine, 4.3 g of 1-(2-bromo-phenyl)-naphthalene, 0.1 g of palladium(II) acetate, 0.1 g of tri-t-butylphosphine, and 1.5 g of sodium t-butoxide, and the mixture was stirred under reflux overnight in a toluene solvent. After the mixture was allowed to cool, a filtrate obtained by filtering was concentrated, to obtain a crude product. The obtained crude product was purified by crystallization with a toluene/acetone mixed solvent, to obtain 5.2 g of a pale-yellow powder of (3',5'-diphenyl-1,1':2',1"-terphenyl-3"-yl)-(2-naphthalen-1-yl-phenyl)-(1,1':4',1"-terphenyl-4-yl)-amine (Compound (136)) (yield: 48.7%).

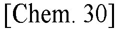

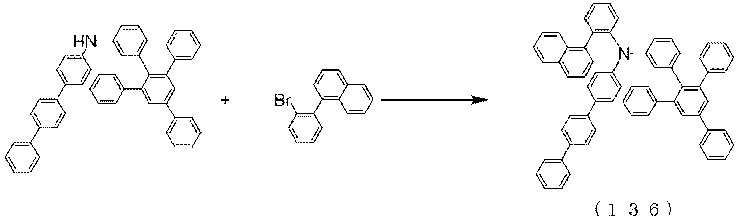

The structure of the obtained pale-yellow powder was identified by NMR.

The following 45 hydrogen signals were detected with ¹H-NMR (CDCl₃).

δ (ppm) = 7.66 (3H), 7.63 (2H), 7.59 (5H), 7.45 (5H), 7.38-7.23 (14H), 7.22-7.11 (6H), 7.01 (1H), 6.89 (1H), 6.78 (3H), 6.66 (1H), 6.56 (1H), 6.43 (1H), 6.09 (2H).

### Example 30:

Using a high-sensitivity differential scanning calorimeter (DSC3100SA from Bruker AXS), the melting point and the glass transition point were measured for each of the arylamine compounds obtained in Examples 1 to 29. The results are shown in Table 1.

**[Table 1]**

| | Melting point | Glass transition point |
|---|---|---|
| Compound of Example 1 | 214°C | 105°C |
| Compound of Example 2 | - | 116°C |
| Compound of Example 3 | - | 112°C |
| Compound of Example 4 | - | 119°C |
| Compound of Example 5 | - | 105°C |
| Compound of Example 6 | - | 114°C |
| Compound of Example 7 | - | 110°C |
| Compound of Example 8 | - | 131°C |
| Compound of Example 9 | - | 121°C |
| Compound of Example 10 | - | 116°C |
| Compound of Example 11 | - | 126°C |
| Compound of Example 12 | - | 136°C |
| Compound of Example 13 | - | 116°C |
| Compound of Example 14 | - | 124°C |
| Compound of Example 15 | - | 124°C |
| Compound of Example 16 | - | 140°C |
| Compound of Example 17 | - | 129°C |
| Compound of Example 18 | - | 133°C |
| Compound of Example 19 | - | 136°C |
| Compound of Example 20 | - | 137°C |
| Compound of Example 21 | - | 146°C |
| Compound of Example 22 | - | 151°C |
| Compound of Example 23 | - | 130°C |
| Compound of Example 24 | - | 130°C |
| Compound of Example 25 | - | 126°C |
| Compound of Example 26 | - | 123°C |
| Compound of Example 27 | - | 117°C |
| Compound of Example 28 | - | 117°C |
| Compound of Example 29 | - | 126°C |

The results show that the arylamine compounds obtained in Examples 1 to 29 each have a glass transition point of 100°C or higher. This indicates that these compounds are stable in a thin-film state.

### Example 31:

A 100-nm-thick vapor deposition film was formed on an ITO substrate by using the respective arylamine compounds obtained in Examples 1 to 27, and the work function was measured using an ionization potential measurement device (PYS-202 from Sumitomo Heavy Industries, Ltd.). The results are shown in Table 2.

**[Table 2]**

| | Work function |
|---|---|
| Compound of Example 1 | 5.76 eV |
| Compound of Example 2 | 5.75 eV |
| Compound of Example 3 | 5.74 eV |
| Compound of Example 4 | 5.83 eV |
| Compound of Example 5 | 5.77 eV |
| Compound of Example 6 | 5.79 eV |
| Compound of Example 7 | 5.77 eV |
| Compound of Example 8 | 5.79 eV |
| Compound of Example 9 | 5.76 eV |
| Compound of Example 10 | 5.72 eV |
| Compound of Example 11 | 5.79 eV |
| Compound of Example 12 | 5.78 eV |
| Compound of Example 13 | 5.77 eV |
| Compound of Example 14 | 5.78 eV |
| Compound of Example 15 | 5.74 eV |
| Compound of Example 16 | 5.78 eV |
| Compound of Example 17 | 5.71 eV |
| Compound of Example 18 | 5.71 eV |
| Compound of Example 19 | 5.69 eV |
| Compound of Example 20 | 5.72 eV |
| Compound of Example 21 | 5.70 eV |
| Compound of Example 22 | 5.72 eV |
| Compound of Example 23 | 5.79 eV |
| Compound of Example 24 | 5.77 eV |
| Compound of Example 25 | 5.78 eV |
| Compound of Example 26 | 5.68 eV |
| Compound of Example 27 | 5.79 eV |
| Compound of Example 28 | 5.74 eV |
| Compound of Example 29 | 5.75 eV |

The results show that the arylamine compounds obtained in Examples 1 to 27 exhibit a more suitable energy level compared to the work function of approximately 5.4 eV of typical hole-transporting materials such as NPD, TPD, etc., and thus have favorable hole transportability.

### Example 32:

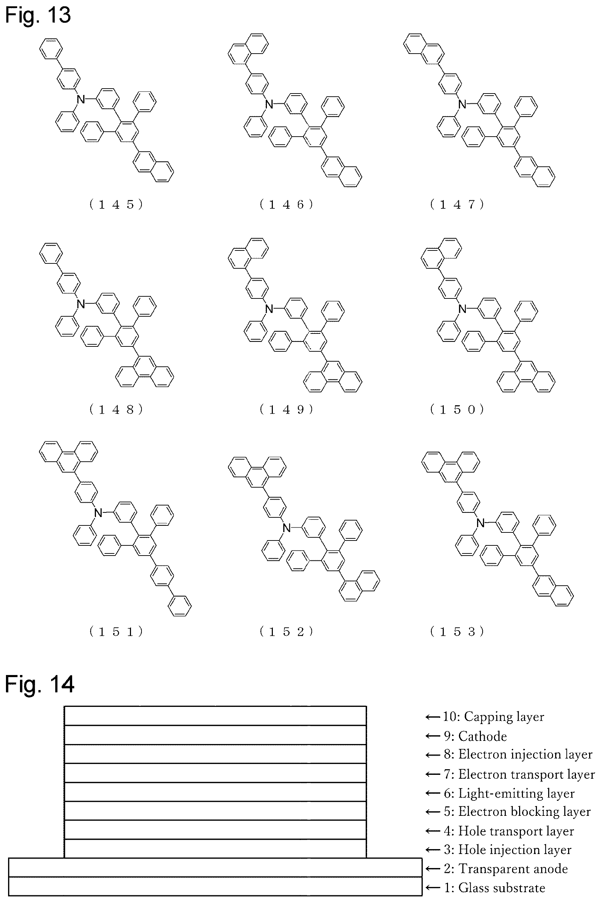

As illustrated in Fig. 13, an organic EL device was prepared by vapor-depositing a hole injection layer 3, a hole transport layer 4, an electron blocking layer 5, a light-emitting layer 6, an electron transport layer 7, an electron injection layer 8, a cathode 9, and a capping layer 10 in this order onto a glass substrate 1 having formed thereon a reflective ITO electrode as a transparent anode 2 in advance.

More specifically, a glass substrate 1 having formed thereon, in order, a 50-nm-thick ITO film, a 100-nm-thick silver-alloy reflective film, and a 5-nm-thick ITO film was subjected to ultrasonic cleaning in isopropyl alcohol for 20 minutes, and then dried for 10 minutes on a hot plate heated to 250°C. Then, after UV ozone treatment for 2 minutes, the glass substrate having the ITO was mounted to a vacuum vapor deposition apparatus, in which the pressure was reduced to 0.001 Pa or lower.

Next, a hole injection layer 3 was formed so as to cover the transparent anode 2 and so that the film thickness was 10 nm, by performing binary vapor deposition of an electron acceptor (Acceptor-1) having the following structural formula and a compound (HTM-1) having the following structural formula at a rate at which the vapor deposition rate ratio between Acceptor-1 and HTM-1 was 3:97.

On this hole injection layer 3, the compound (HTM-1) having the following structural formula was formed as a hole transport layer 4 having a film thickness of 140 nm.

On this hole transport layer 4, Compound (58) of Example 1 was formed as an electron blocking layer 5 having a film thickness of 5 nm.

On this electron blocking layer 5, a light-emitting layer 6 was formed so that the film thickness was 20 nm, by performing binary vapor deposition of a compound (EMD-1) having the following structural formula and a compound (EMH-1) having the following structural formula at a rate at which the vapor deposition rate ratio between EMD-1 and EMH-1 was 5:95.

On this light-emitting layer 6, an electron transport layer 7 was formed so that the film thickness was 30 nm, by performing binary vapor deposition of a compound (ETM-1) having the following structural formula and a compound (ETM-2) having the following structural formula at a rate at which the vapor deposition rate ratio between ETM-1 and ETM-2 was 50:50.

On this electron transport layer 7, lithium fluoride was formed as an electron injection layer 8 having a film thickness of 1 nm.

On this electron injection layer 8, a magnesium-silver alloy was formed as a cathode 9 having a film thickness of 12 nm.

Finally, a compound (CPL-1) having the following structure was formed as a capping layer 10 having a film thickness of 60 nm.

Properties of the produced organic EL device were measured in the atmosphere at atmospheric temperature. Measurement results of light emission properties when a direct-current voltage was applied to the produced organic EL device are collectively shown in Table 3.

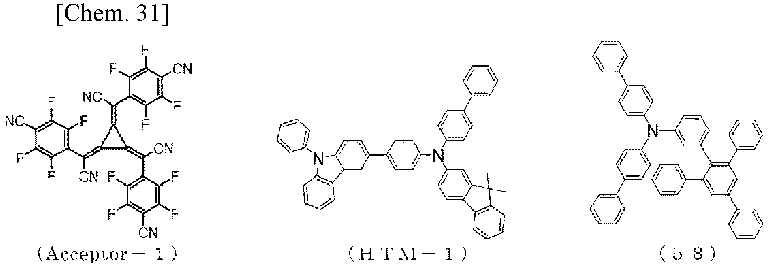

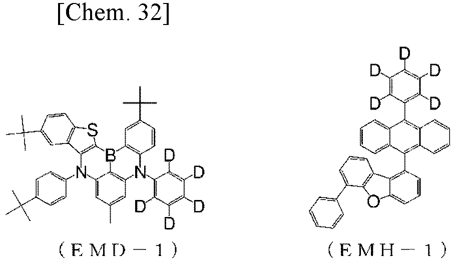

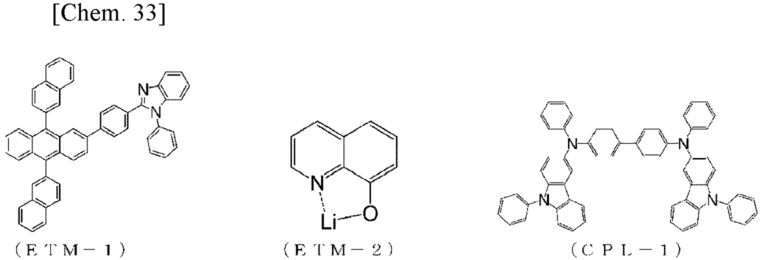

### Example 33:

An organic EL device was produced according to the same conditions, except that, in Example 32, Compound (59) of Example 2 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 34:

An organic EL device was produced according to the same conditions, except that, in Example 32, Compound (60) of Example 3 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 35:

An organic EL device was produced according to the same conditions, except that, in Example 32, Compound (15) of Example 4 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 36:

An organic EL device was produced according to the same conditions, except that, in Example 32, Compound (31) of Example 5 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 37:

An organic EL device was produced according to the same conditions, except that, in Example 32, Compound (32) of Example 6 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 38:

An organic EL device was produced according to the same conditions, except that, in Example 32, Compound (33) of Example 7 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 39:

An organic EL device was produced according to the same conditions, except that, in Example 32, Compound (61) of Example 8 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 40:

An organic EL device was produced according to the same conditions, except that, in Example 32, Compound (76) of Example 9 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 41:

An organic EL device was produced according to the same conditions, except that, in Example 32, Compound (77) of Example 10 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 42:

An organic EL device was produced according to the same conditions, except that, in Example 32, Compound (78) of Example 11 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 43:

An organic EL device was produced according to the same conditions, except that, in Example 32, Compound (79) of Example 12 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 44:

An organic EL device was produced according to the same conditions, except that, in Example 32, Compound (96) of Example 13 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 45:

An organic EL device was produced according to the same conditions, except that, in Example 32, Compound (100) of Example 14 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 46:

An organic EL device was produced according to the same conditions, except that, in Example 32, Compound (101) of Example 15 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 47:

An organic EL device was produced according to the same conditions, except that, in Example 32, Compound (102) of Example 16 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 48:

An organic EL device was produced according to the same conditions, except that, in Example 32, Compound (107) of Example 17 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 49:

An organic EL device was produced according to the same conditions, except that, in Example 32, Compound (115) of Example 18 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 50:

An organic EL device was produced according to the same conditions, except that, in Example 32, Compound (116) of Example 19 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 51:

An organic EL device was produced according to the same conditions, except that, in Example 32, Compound (117) of Example 20 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 52:

An organic EL device was produced according to the same conditions, except that, in Example 32, Compound (118) of Example 21 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 53:

An organic EL device was produced according to the same conditions, except that, in Example 32, Compound (119) of Example 22 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 54:

An organic EL device was produced according to the same conditions, except that, in Example 32, Compound (127) of Example 23 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 55:

An organic EL device was produced according to the same conditions, except that, in Example 32, Compound (130) of Example 24 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 56:

An organic EL device was produced according to the same conditions, except that, in Example 32, Compound (132) of Example 25 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 57:

An organic EL device was produced according to the same conditions, except that, in Example 32, Compound (133) of Example 26 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 58:

An organic EL device was produced according to the same conditions, except that, in Example 32, Compound (134) of Example 27 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 59:

An organic EL device was produced according to the same conditions, except that, in Example 32, Compound (135) of Example 28 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Example 60:

An organic EL device was produced according to the same conditions, except that, in Example 32, Compound (136) of Example 29 was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

### Comparative Example 1:

For comparison, an organic EL device was produced according to the same conditions, except that, in Example 32, a compound (HTM-2) having the following structural formula (see, for example, Patent Literature 5) was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

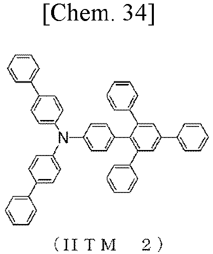

### Comparative Example 2:

For comparison, an organic EL device was produced according to the same conditions, except that, in Example 32, a compound (HTM-3) having the following structural formula was used instead of Compound (58) of Example 1 as the material for the electron blocking layer 5. Properties of the produced organic EL device were measured in the atmosphere at atmospheric temperature. Measurement results of light emission properties when a direct-current voltage was applied to the produced organic EL device are collectively shown in Table 3.

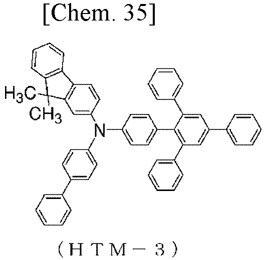

Device life was measured using the organic EL devices produced in Examples 32 to 60 and Comparative Examples 1 and 2. The results are collectively shown in Table 3. Device life was measured as follows. Constant current driving was performed, with the light emission luminance at the start of light emission (i.e., initial luminance) being 1000 cd/m², and the time it took for the light emission luminance to attenuate to 950 cd/m² (95% attenuation: amounting to 95% when the initial luminance is considered 100%) was measured.

**[Table 3]**

| | Electron blocking layer | (@10 mA/cm²) | | | | Device life |
|---|---|---|---|---|---|---|
| | | Voltage [V] | Luminance [cd/m²] | Luminous efficiency | Power efficiency | 95% attenuation |
| Example 32 | Compound 58 | 3.45 | 1011 | 10.11 | 9.21 | 282 |
| Example 33 | Compound 59 | 3.47 | 1001 | 10.01 | 9.18 | 311 |
| Example 34 | Compound 60 | 3.43 | 1036 | 10.36 | 9.40 | 365 |
| Example 35 | Compound 15 | 3.46 | 940 | 9.40 | 8.51 | 339 |
| Example 36 | Compound 31 | 3.44 | 1005 | 10.06 | 9.20 | 283 |
| Example 37 | Compound 32 | 3.47 | 1022 | 10.24 | 9.28 | 294 |
| Example 38 | Compound 33 | 3.44 | 1022 | 10.23 | 9.36 | 378 |
| Example 39 | Compound 61 | 3.46 | 940 | 9.40 | 8.51 | 339 |
| Example 40 | Compound 76 | 3.46 | 1070 | 10.71 | 9.74 | 276 |
| Example 41 | Compound 77 | 3.43 | 1018 | 10.18 | 9.34 | 443 |
| Example 42 | Compound 78 | 3.49 | 1102 | 11.05 | 9.95 | 291 |
| Example 43 | Compound 79 | 3.48 | 1027 | 10.27 | 9.27 | 267 |
| Example 44 | Compound 96 | 3.46 | 954 | 9.55 | 8.67 | 272 |
| Example 45 | Compound 100 | 3.39 | 1052 | 10.52 | 9.76 | 310 |
| Example 46 | Compound 101 | 3.41 | 994 | 9.94 | 9.14 | 348 |
| Example 47 | Compound 102 | 3.41 | 1056 | 10.56 | 9.74 | 285 |
| Example 48 | Compound 107 | 3.34 | 1067 | 10.67 | 9.61 | 434 |
| Example 49 | Compound 115 | 3.43 | 964 | 9.64 | 8.83 | 401 |
| Example 50 | Compound 116 | 3.42 | 1041 | 10.42 | 9.58 | 632 |
| Example 51 | Compound 117 | 3.41 | 1024 | 10.24 | 9.44 | 295 |
| Example 52 | Compound 118 | 3.44 | 1002 | 10.02 | 9.16 | 284 |
| Example 53 | Compound 119 | 3.47 | 943 | 9.43 | 8.54 | 272 |
| Example 54 | Compound 127 | 3.47 | 1040 | 10.44 | 9.45 | 275 |
| Example 55 | Compound 130 | 3.47 | 1058 | 10.59 | 9.60 | 306 |
| Example 56 | Compound 132 | 3.38 | 986 | 9.86 | 9.10 | 389 |
| Example 57 | Compound 133 | 3.43 | 937 | 9.37 | 8.61 | 363 |
| Example 58 | Compound 134 | 3.40 | 987 | 9.87 | 9.13 | 292 |
| Example 59 | Compound 135 | 3.44 | 1014 | 10.15 | 9.29 | 353 |
| Example 60 | Compound 136 | 3.43 | 1011 | 10.12 | 9.24 | 311 |
| Comparative | HTM-2 | 3.49 | 915 | 9.15 | 8.23 | 245 |
| Comparative | HTM-3 | 3.54 | 897 | 8.97 | 8.19 | 269 |

As shown in Table 3, the organic EL devices (Examples 33, 34, 40, 41, and 42) employing the arylamine compounds of the present invention (compounds 59, 60, 76, 77, and 78) have low driving voltages. Further, while the luminous efficiency when a current having a current density of 10 mA/cm² was passed was 8.97 to 9.15 cd/A for the organic EL devices of Comparative Examples 1 and 2, the organic EL devices of Examples 32 to 60 had high efficiency of 9.37 to 11.05 cd/A. Furthermore, while the organic EL devices of Comparative Examples 1 and 2 had a power efficiency of 8.19 to 8.23 lm/W, the organic EL devices of Examples 32 to 60 had high efficiency of 8.51 to 9.95 lm/W. Furthermore, while the organic EL devices of Comparative Examples 1 and 2 had a device life (95% attenuation) of 245 to 269 hours, the organic EL devices of Examples 32 to 60 had comparable or longer lifetime of 267 to 632 hours.

These results reveal that, since the organic EL devices of the present invention use arylamine compounds having high hole mobility and excellent electron blockability, it is possible to achieve organic EL devices having higher luminous efficiency and longer lifetime while maintaining low driving voltage, compared to conventional organic EL devices.

### Industrial Applicability

The organic EL device according to the present invention, which uses an arylamine compound having a specific structure, has improved luminous efficiency, and also, the organic EL device can be improved in durability. Thus, for example, application can be expanded to home electrical appliances and lightings. Further, the arylamine compound of the present invention is not only usable for organic EL devices, but also usable in the field of electronic devices, such as electrophotographic photoreceptors, image sensors, photoelectric conversion devices, solar cells, etc.

1: Glass substrate
2: Transparent anode
3: Hole injection layer
4: Hole transport layer
5: Electron blocking layer
6: Light-emitting layer
7: Electron transport layer
8: Electron injection layer
9: Cathode
10: Capping layer

## Claims

1. An arylamine compound selected from the following compounds: 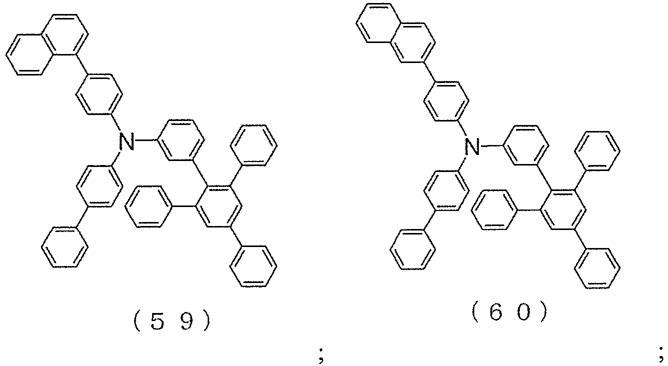 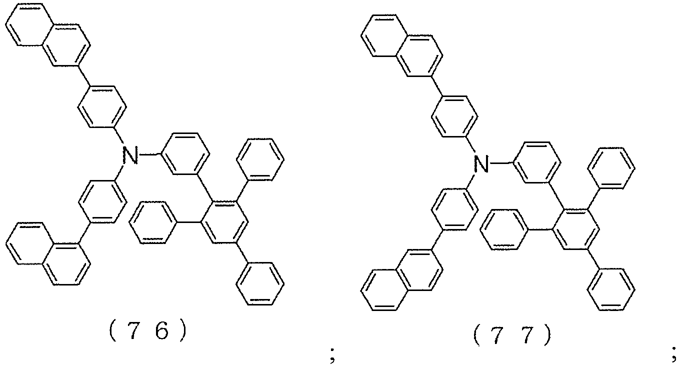 and

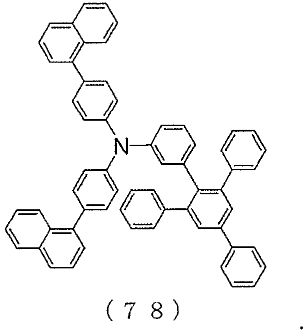

2. An organic electroluminescence device comprising:
a pair of electrodes (2, 9); and
at least one organic layer (3 to 8) sandwiched between the electrodes, wherein
the organic layer contains the arylamine compound according to claim 1.

3. The organic electroluminescence device according to claim 2, wherein the organic layer is a hole transport layer.

4. The organic electroluminescence device according to claim 2, wherein the organic layer is an electron blocking layer.

5. The organic electroluminescence device according to claim 2, wherein the organic layer is a hole injection layer.

6. The organic electroluminescence device according to claim 2, wherein the organic layer is a light-emitting layer.

7. An electronic device employing an electronic component comprising:
a pair of electrodes; and
at least one organic layer sandwiched between the electrodes, wherein
the organic layer contains the arylamine compound according to claim 1.

## Patentansprüche

1. Arylaminverbindung, ausgewählt aus den folgenden Verbindungen: 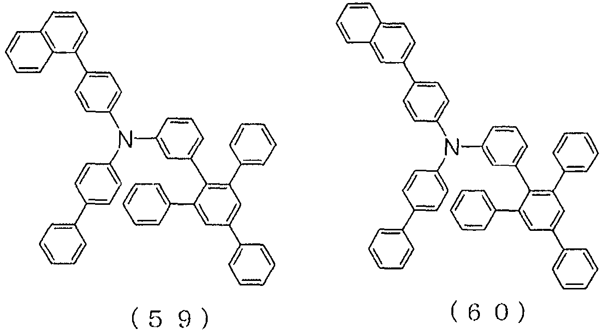 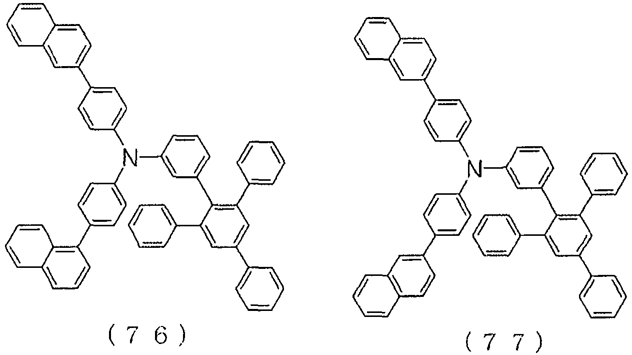 und

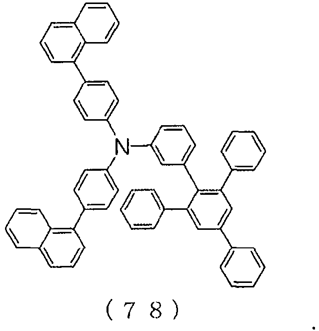

2. Organische Elektrolumineszenzvorrichtung, umfassend:
ein Paar Elektroden (2, 9); und
mindestens eine organische Schicht (3 bis 8), die zwischen den Elektroden angeordnet ist, wobei
die organische Schicht die Arylaminverbindung nach Anspruch 1 enthält.

3. Organische Elektrolumineszenzvorrichtung nach Anspruch 2, wobei die organische Schicht eine Lochtransportschicht ist.

4. Organische Elektrolumineszenzvorrichtung nach Anspruch 2, wobei die organische Schicht eine Elektronenblockierschicht ist.

5. Organische Elektrolumineszenzvorrichtung nach Anspruch 2, wobei die organische Schicht eine Lochinjektionsschicht ist.

6. Organische Elektrolumineszenzvorrichtung nach Anspruch 2, wobei die organische Schicht eine lichtemittierende Schicht ist.

7. Elektronische Vorrichtung, die eine elektronische Komponente verwendet, umfassend:
ein Paar Elektroden; und
mindestens eine zwischen den Elektroden angeordnete organische Schicht, wobei
die organische Schicht die Arylaminverbindung nach Anspruch 1 enthält.

## Revendications

1. Composé d'arylamine choisi parmi les composés suivants: 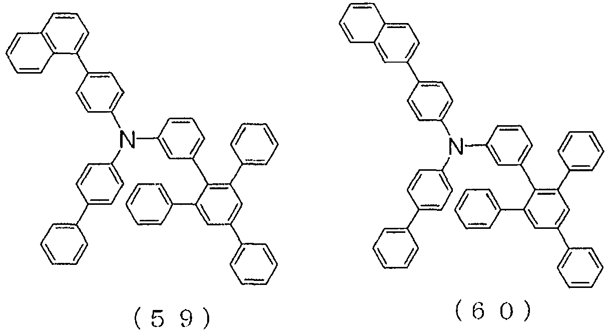 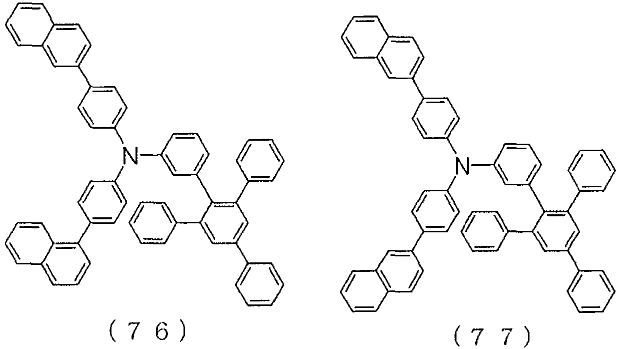 et

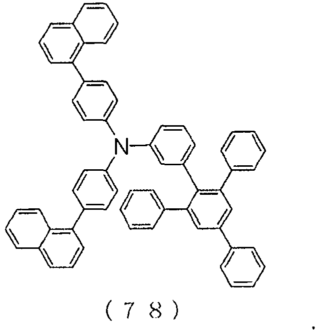

2. Dispositif électroluminescent organique comprenant :
une paire d'électrodes (2, 9) ; et
au moins une couche organique (3 à 8) prise en sandwich entre les électrodes, dans lequel
la couche organique contient le composé d'arylamine selon la revendication 1.

3. Dispositif électroluminescent organique selon la revendication 2, dans lequel la couche organique est une couche de transport de trous.

4. Dispositif électroluminescent organique selon la revendication 2, dans lequel la couche organique est une couche de blocage d'électrons.

5. Dispositif électroluminescent organique selon la revendication 2, dans lequel la couche organique est une couche d'injection de trous.

6. Dispositif électroluminescent organique selon la revendication 2, dans lequel la couche organique est une couche émettrice de lumière.

7. Dispositif électronique utilisant un composant électronique comprenant :
une paire d'électrodes ; et
au moins une couche organique prise en sandwich entre les électrodes, dans lequel
la couche organique contient le composé d'arylamine selon la revendication 1.
